(19) Europäisches Patentamt

European Patent Office

Office européen des brevets

(11) **EP 2 507 056 B1**

(12) **EUROPEAN PATENT SPECIFICATION**

(45) Date of publication and mention of the grant of the patent:
**27.03.2019 Bulletin 2019/13**

(21) Application number: **10834004.3**

(22) Date of filing: **29.11.2010**

(51) Int Cl.:
*C08J 7/04* (2006.01)     *C08J 7/18* (2006.01)
*C23C 16/40* (2006.01)    *C23C 16/50* (2006.01)
*C23C 16/54* (2006.01)    *A61K 9/70* (2006.01)
*A61M 35/00* (2006.01)

(86) International application number:
**PCT/US2010/058155**

(87) International publication number:
**WO 2011/066493 (03.06.2011 Gazette 2011/22)**

(54) **CONFORMABLE BARRIER SHEET**

ANPASSUNGSFÄHIGE BARRIEREFOLIE

FEUILLE BARRIERE CONFORMABLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.11.2009 US 265067 P**

(43) Date of publication of application:
**10.10.2012 Bulletin 2012/41**

(73) Proprietor: **3M Innovative Properties Company
St. Paul, MN 55133-3427 (US)**

(72) Inventors:
• **KLUGE, Bruce D.
Saint Paul, Minnesota 55133-3427 (US)**
• **BEST, William G.
Saint Paul, Minnesota 55133-3427 (US)**
• **DAVID, Moses M.
Saint Paul, Minnesota 55133-3427 (US)**
• **KOLB, William Blake
Saint Paul, Minnesota 55133-3427 (US)**
• **MANALO, Daniel O.
Saint Paul, Minnesota 55133-3427 (US)**
• **GORDON, Ryan D.
Saint Paul, Minnesota 55133-3427 (US)**

(74) Representative: **Vossius & Partner
Patentanwälte Rechtsanwälte mbB
Siebertstrasse 3
81675 München (DE)**

(56) References cited:
**US-A1- 2004 219 198     US-A1- 2007 020 451
US-B1- 6 881 538**

Remarks:
The file contains technical information submitted after the application was filed and not included in this specification

EP 2 507 056 B1

**Description**

BACKGROUND

Packaging and backing materials have been developed which provide an effective barrier to oxygen, water vapor, other gases, as well as other diffusing materials. In one example, certain transdermal drug delivery devices include a backing material which limits moisture and oxygen transmission through the device, as well as limiting diffusion of components of a drug formulation into or through the backing material. In some cases, such packaging and backing materials have typically included metal foils, for example, aluminum foil, as a barrier layer, based upon their resistance to temperature and humidity extremes. Where transparency or translucency has been important, certain inorganic oxides and nitrides, for example, silicon oxide, aluminum oxide, aluminum-silicon-oxide, indium-tin-oxide, aluminum-silicon-oxy-nitride, and magnesium oxide have been proposed as a barrier layer on a film, using vapor deposition or sputtering methods to form such layers.

These barrier layers have been provided on a polyester substrate, because of its heat stability, mechanical and dimensional stability, including resistance to stretching after the barrier layer is applied. However, in certain applications, including, for example, packaging and transdermal drug delivery devices, greater flexibility may be desired.

U.S. Patent Application Publication 2007/0020451 A1 discloses a barrier sheet comprising a polymeric film, a planarization layer, and a plasma-deposited amorphous glass layer, wherein the planarization layer is disposed on the polymeric film, and the amorphous glass layer is deposited on the planarization layer. The polymeric film may for example be made of high density polyethylene, low density polyethylene, polypropylene, or a polyurethane. The planarization layer can be obtained by coating the polymeric film with multifunctional (meth)acrylate monomers and subsequent cross-linking, and preferably has a thickness of 0.050 to 0.130 $\mu$m, more preferably of 0.065 to 0.100 $\mu$m. The amorphous glass layer can be obtained by plasma enhanced chemical vapour deposition (PECVD) from a source gas comprising an organosilicon compound such as tetramethylsilane and oxygen, and preferably has a thickness of at least 0.100 $\mu$m. It comprises, on a hydrogen-free basis, at least 25% silicon, at least 30% carbon, and no more than 45% oxygen, and may optionally further comprise hydrogen. Said barrier sheet may for example be used for enveloping an article.

There is a continuing need for materials and devices which provide effective barrier properties while providing greater flexibility.

SUMMARY

The present invention is as defined in the appended claims. Generally, conformable barrier sheets have been found, which include a substrate having a significant degree of stretchability, a planarization layer, and a plasma-deposited amorphous glass layer, and which can be stretched, such as during processing and use, without appreciable loss of moisture vapor transmission resistance.

Accordingly, in one disclosed embodiment, there is provided a conformable barrier sheet comprising:

a polymeric film having a tensile elongation at least 2 fold greater than a polyethylene terephthalate film of equal dimensions and under an equal load;
a planarization layer; and
a plasma-deposited amorphous glass layer comprising silicon, carbon, and hydrogen;
wherein the planarization layer is disposed on the polymeric film, the amorphous glass layer is deposited on the planarization layer, and
wherein the barrier sheet is sufficiently conformable that after undergoing a tensile elongation within the range of more than 2 % to 20 %, the barrier sheet has a moisture vapor transmission rate essentially unchanged compared with prior to being elongated.

In another disclosed embodiment, there is provided a method of manufacturing a conformable barrier sheet, the method comprising:

providing a polymeric film having an elongation at least 2 fold greater than a polyethylene terephthalate film of equal dimensions and under an equal load;
forming a planarization layer on the polymeric film; and
plasma-depositing an amorphous glass layer comprising silicon, carbon, and hydrogen onto the planarization layer;
wherein the barrier sheet is sufficiently conformable that after undergoing a tensile elongation within the range of more than 2 % to 20 %, the barrier sheet has a moisture vapor transmission rate essentially unchanged compared with prior to being elongated.

In another disclosed embodiment, there is provided a transdermal drug delivery device comprising:

a conformable barrier sheet comprising:

a polymeric film having a tensile elongation at least 2 fold greater than a polyethylene terephthalate film of equal dimensions and under an equal load;
a planarization layer; and
a plasma-deposited amorphous glass layer comprising silicon, carbon, and hydrogen;
wherein the planarization layer is disposed on the polymeric film, the amorphous glass layer is deposited on the planarization layer, and
wherein the barrier sheet is sufficiently conformable that after undergoing a tensile elongation within the range of more than 2 % to 20 %, the barrier sheet has a moisture vapor transmission rate essentially unchanged compared with prior to being elongated; and

a reservoir adjoining the polymeric film or the amorphous glass layer, the reservoir comprising a releasably stored dosage of a pharmaceutically active agent.

In another disclosed embodiment , there is provided a method of delivering a drug to a mammal comprising;

providing a transdermal drug delivery device according to the above transdermal drug delivery device embodiment or any one embodiment thereof described herein;
placing a surface of the reservoir directly adjoining skin of the mammal; and
allowing the reservoir to remain directly adjoining the skin for a period of time sufficient to provide a therapeutic effect.

In another disclosed embodiment, there is provided a method of protecting an article comprising enveloping the article with a protective covering comprising any one of the embodiments of the conformable high barrier sheet described herein.

DEFINITIONS

As used herein, the term "essentially unchanged" with respect to moisture vapor transmission rate or oxygen transmission rate refers to less than a 2 fold increase, preferably less than a 1.5 fold, more preferably less than a 1.1 fold increase, most preferably no increase after undergoing a tensile elongation within the range of more than 2 % to 20 %.
The term "comprising" and variations thereof (e.g., comprises, includes, etc.) do not have a limiting meaning where these terms appear in the description and claims.
As used herein, "a," "an," "the," "at least one," and "one or more" are used interchangeably, unless the context clearly dictates otherwise.
Also herein, the recitations of numerical ranges by endpoints include all numbers subsumed within that range (e.g., 12.7 to 127 micrometers includes 12.7, 12.9, 15, 50.8, 76.2, 100, 101.6, 127, etc.).
The above summary of the present disclosure is not intended to describe each disclosed embodiment or every implementation of the present disclosure. The Figures and the detailed description that follow more particularly exemplify illustrative embodiments.

BRIEF DESCRIPTION OF THE DRAWINGS

FIG. 1 shows a schematic cross-section of a conformable barrier sheet used in the present invention.
FIG. 2 shows a schematic cross-section of a transdermal drug delivery device embodiment of the present invention where a reservoir directly adjoins an amorphous glass layer.
FIG. 3 shows a schematic cross-section of a transdermal drug delivery device embodiment of the present invention where the reservoir directly adjoins a polymeric film to which a plasma-deposited amorphous glass layer has been applied on the opposite side of the film.

DETAILED DESCRIPTION OF ILLUSTRATIVE EMBODIMENTS

A conformable, transparent or translucent sheet material with durable, high barrier properties, which can be used for packaging, for example, as a protective covering for a product sensitive to moisture and/or oxygen until now has not been available. Although available conformable substrates are sufficiently stretchy to provide the desired conformability, previous barrier layers associated with such conformable substrates have been found to crack, delaminate, or otherwise lose a substantial amount of their barrier properties, for example, moisture vapor transmission resistance, when the substrate is stretched.

A conformable high barrier sheet comprising a polymeric film backing layer, a planarization layer, and an amorphous glass layer has now been found which provides substantial elongation without loosing resistance to water vapor transmission. The substantial elongation along with the high barrier properties provide conformability and a stable environment for a moisture and/or oxygen sensitive product packaged with the barrier sheet. In another example, the conformable high barrier sheet provides a stable environment for a drug, making the sheet well suited for uses such as for a conformable backing material in devices, including transdermal drug delivery devices. Moreover, the ability to maintain resistance to water vapor transmission after being placed under tension, causing elongation of the sheet, allows web handling of the barrier sheet required for fabricating such devices, as well as any applications where the barrier sheet is stretched.

As indicated above, the polymeric film has a tensile elongation at least 2 fold greater than a polyethylene terephthalate (PET) film of equal dimensions and under an equal load. PET films, which have been used in packaging and transdermal drug delivery devices, while flexible in the sense that they bend, have relatively low tensile elongation and have been found not to feel as conformable in such devices as films having greater elongation. For certain embodiments, including any one of the above embodiments, preferably the polymeric film has a tensile elongation at least 3 fold greater than a PET film of equal dimensions and under an equal load. For certain of these embodiments, preferably the tensile elongation is at least 4 fold, more preferably at least 6 fold greater than a PET film of equal dimensions and under an equal load. For purposes of this comparison, the PET film consists only of polyethylene terephthalate except for any additives that may be used in the film (e.g., slip agents, etc.). The most common PET films are biaxially stretched PET with a density of about 1.39 g/cm$^3$. Such films are available commercially and include HOSTAPHAN (Mitsubishi Polyester Film GmbH), MYLAR (DuPont Teijin Films), MELINEX (DuPont Teijin Films), SCOTCHPAK 9753 (3M Company, St. Paul, MN), and the like. For purposes of comparative testing, SCOTCHPAK 9753 was used herein.

For certain embodiments, including any one of the above embodiments, the polymeric film has an elongation of at least 5 percent when a 9 inch (22.9 cm) width and 6 inch (15.2 cm) length of the film having a thickness of 0.003 inches (76.2 micrometers) is subjected to a 25 pound (11.3 kg) load across its full width. For certain of these embodiments, preferably the elongation is at least 10 percent, preferably at least 15 percent, more preferably at least 20 percent, at a 30 pound (13.6 kg) load.

For certain embodiments, including any one of the above embodiments, the polymeric film has an elongation without breaking of at least 100 % when tested at a thickness of 12.7 to 127 micrometers. For certain of these embodiments, the polymeric film has an elongation of at least 130 % and a maximum elongation of 600 percent.

The planarization layer is disposed on and covers a major surface of the polymeric film of the barrier sheet. The planarization layer, in certain embodiments, is sufficiently thick to be a continuous layer, such that both high and low areas in the topography on the polymeric film surface are covered. For certain embodiments, including any one of the above embodiments, preferably the planarization layer has a thickness greater than 1.2 micrometers. Lower thicknesses have now been found to provide less effective barrier properties when the barrier sheet is subjected to stresses resulting in appreciable film elongation. For certain of these embodiments, preferably the planarization layer has a thickness of at least 1.5 micrometers. For certain of these embodiments, preferably the planarization layer has a thickness of at least 2 micrometers. For certain of these embodiments, the thickness is not greater than 6 micrometers, more preferably not greater than 4 micrometers. Higher thicknesses have been found to be unnecessary and, in certain instances, counterproductive for maintaining low moisture vapor transmission through a stressed barrier sheet.

Using a surface analytic technique such as ESCA, the elemental composition of the plasma-deposited amorphous glass layer can be determined on a hydrogen free basis, although hydrogen is present in the layer. For certain embodiments, including any one of the above embodiments, the amorphous glass layer on a hydrogen-free basis comprises about 20 to 40 atomic percent silicon and greater than 35 atomic percent carbon. For certain of these embodiments, the amorphous glass layer further comprises less than 45 down to and including zero atomic percent oxygen. When sufficient oxygen is present, for example, greater than 25 atomic percent oxygen, the barrier sheet has very low color and appears clear and colorless. For certain of these embodiments, the barrier sheet is essentially colorless.

Alternatively, for certain embodiments, including any one of the above embodiments except where the amorphous glass layer has low or no color, the amorphous glass layer on a hydrogen-free basis comprises about 20 to 40 atomic percent silicon and greater than 50 atomic percent carbon, and further comprises less than 25 down to and including zero atomic percent oxygen. When a low level of oxygen or no oxygen is present, the barrier sheet has a brown color, for example, a tan color or a gold color. This may be preferred when used in a device where such a color may be preferred, such as on a subject having tan skin. For certain of these embodiments, the barrier sheet is a brown color.

For effective barrier properties, in certain embodiments, preferably the amorphous glass layer has a thickness of at least 0.05 micrometer, more preferably at least 0.075 micrometer, most preferably at least 0.1 micrometer. For certain of these embodiments, the thickness is at most 0.5 micrometer, preferably at most 0.4, more preferably at most 0.3 micrometer. For certain embodiments, including any one of the above embodiments, the amorphous glass layer has a thickness of about 0.1 micrometer to about 0.5 micrometer. For certain of these embodiments, the amorphous glass layer has a thickness of 0.1 to 0.3 micrometer. For certain of these embodiments, the amorphous glass layer has a thickness of 0.1 to 0.2 micrometer. When low or no color is desired, the lower thicknesses may be used, and when a brown color is desired, a relatively higher thickness may be used.

The polymeric film used in the present conformable barrier sheet may be a single layer or a multilayer construction. Whether a single layer or a layer in a multilayer construction, the layer may be composed of a single polymer or a blend of two or more polymers. Any given polymer may be made by polymerizing one or more monomers, to provide, for example, a homopolymer, a copolymer, a terpolymer, or a polymer comprising even more than three different monomer precursors. Moreover, each layer in a multilayer construction may be comprised of the same polymer or blend of polymers or a different polymer or a different blend of polymers.

For certain embodiments, including any one of the above embodiments, the polymeric film is selected from the group consisting of a single layer film and a multilayer film.

For certain embodiments, including any one of the above embodiments, the polymeric film comprises a polymer selected from the group consisting high density polyethylene, medium density polyethylene, low density polyethylene, very low density polyethylene, linear low density polyethylene, ultra low density polyethylene, polypropylene, poly(ethylene-co-propylene), poly(ethylene-co-hexene), poly(ethylene-co-octene), poly(ethylene-co-butene), poly(ethylene-co-vinyl acetate), poly(ethylene-co-vinyl alcohol), poly(ethylene-co-acrylic acid), a polyurethane, a thermoplastic polyester elastomer (available under the trade name HYTREL from E.I. du Pont de Nemours and Company), a blend thereof, and a combination thereof. For certain embodiments, including any one of the above embodiments, the polymeric film comprises a polymer selected from the group consisting high density polyethylene, medium density polyethylene, low density polyethylene, very low density polyethylene, linear low density polyethylene, ultra low density polyethylene, polypropylene, poly(ethylene-co-propylene), poly(ethylene-co-hexene), poly(ethylene-co-octene), poly(ethylene-co-butene), poly(ethylene-co-vinyl acetate), poly(ethylene-co-vinyl alcohol), poly(ethylene-co-acrylic acid), a polyurethane, a blend thereof, and a combination thereof. For certain of these embodiments, the polymeric film is selected from the group consisting of poly(ethylene-co-vinyl acetate) with a vinyl acetate content of 2 to 32 percent by weight, a blend of ultra low density polyethylene and linear low density polyethylene, low density polyethylene, a polyurethane, and a thermoplastic polyester elastomer. For certain of these embodiments, the polymeric film is selected from the group consisting of poly(ethylene-co-vinyl acetate) with a vinyl acetate content of 2 to 32 percent by weight, a blend of ultra low density polyethylene and linear low density polyethylene, low density polyethylene, and polyurethane. For certain of these embodiments, the polymeric film is poly(ethylene-co-vinyl acetate) with a vinyl acetate content of 2 to 32 percent by weight. For certain of these embodiments, the vinyl acetate content is 2 to 25 percent by weight, preferably 2 to 19 percent by weight.

The polymeric film has a thickness sufficient to support the planarization and amorphous glass layers and provide sufficient strength for handling. On the other hand, the film is sufficiently thin that the desired conformability is maintained. For certain embodiments, including any one of the above embodiments, the polymeric film has a thickness of no more than about 150 micrometers, preferably not more than 125, more preferably not more than 100 micrometers. For certain embodiments, including any one of the above embodiments, the polymeric film has a thickness of at least about 10 micrometers, preferably at least 25 micrometers.

The planarization layer provides a relatively smooth surface upon which the amorphous glass layer is applied. The planarization layer can be formed on a major surface of the polymeric film by coating a solution of at least one cross-linkable monomer using conventional coating methods such as roll coating (e.g., gravure roll coating), spray coating (e.g., electrostatic spray coating), curtain coat, die coating, and the like and then cross-linking by exposure to visible, ultraviolet, and/or electron beam radiation. For certain embodiments, including any one of the above embodiments, the planarization layer is a cross-linked polymer.

Preferred monomers include multifunctional (meth)acrylates used alone or in combination with other multifunctional or monofunctional (meth)acrylates. Examples of suitable monomers include, but are not limited to, hexanediol diacrylate, ethoxyethyl acrylate, phenoxyethyl acrylate, cyanoethyl (mono)acrylate, isobornyl acrylate, isobornyl methacrylate, octadecyl acrylate, isodecyl acrylate, lauryl acrylate, beta-carboxyethyl acrylate, tetrahydrofurfuryl acrylate, dinitrile acrylate, pentafluorophenyl acrylate, nitrophenyl acrylate, 2-phenoxyethyl acrylate, 2-phenoxyethyl methacrylate, 2,2,2-trifluoromethyl (meth)acrylate, diethylene glycol diacrylate, triethylene glycol diacrylate, triethylene glycol dimethacrylate, tripropylene glycol diacrylate, tetraethylene glycol diacrylate, neopentyl glycol diacrylate, propoxylated neopentyl glycol diacrylate, polyethylene glycol diacrylate, tetraethylene glycol diacrylate, bisphenol A epoxy diacrylate, 1,6-hexanediol dimethacrylate, trimethylolpropane triacrylate, trimethylolpropane ethoxylate triacrylate, trimethylolpropane propoxylate triacrylate, trimethylolpropane trimethacrylate, trimethylolpropane ethoxylate trimethacrylate, trimethylolpropane propoxylate trimethacrylate, tris(2-hydroxyethyl)-isocyanurate triacrylate, tris(2-hydroxyethyl)-isocyanurate trimethacrylate, pentaerythritol triacrylate, pentaerythritol tetraacrylate, pentaerythritol trimethacrylate, pentaerythritol tetramethacrylate, phenylthioethyl acrylate, naphthloxyethyl acrylate, IRR-214 cyclic diacrylate from UCB Chemicals, epoxy acrylate RDX80095 from Rad-Cure Corporation, and mixtures thereof.

For certain embodiments, including any one of the above embodiments for manufacturing a conformable barrier sheet, forming the planarization layer on the polymeric film comprises: coating a cross-linkable composition onto the polymeric film, wherein the cross-linkable composition comprises at least one component having two or more ethylenically unsaturated groups; and cross-linking the cross-linkable composition. For certain of these embodiments, the at least one component having two or more ethylenically unsaturated groups is a multifunctional (meth)acrylate.

For certain embodiments, including any one of the above embodiments where the planarization layer is a cross-linked polymer, the cross-linked polymer comprises a cross-linked multifunctional (meth)acrylate.

For certain of these embodiments, the multifunctional (meth)acrylate is selected from the group consisting of trimethylolpropane triacrylate, trimethylolpropane ethoxylate triacrylate, trimethylolpropane propoxylate triacrylate, trimethylolpropane trimethacrylate, trimethylolpropane ethoxylate trimethacrylate, trimethylolpropane propoxylate trimethacrylate, tris(2-hydroxyethyl)-isocyanurate triacrylate, tris(2-hydroxyethyl)-isocyanurate trimethacrylate, pentaerythritol triacrylate, pentaerythritol tetraacrylate, pentaerythritol trimethacrylate, pentaerythritol tetramethacrylate, and a combination thereof. For certain of these embodiments, the multifunctional (meth)acrylate is pentaerythritol tetraacrylate.

Suitable solvents for the cross-linkable monomer solution include low viscosity (meth)acrylate monomers and volatile solvents such as ethanol, isopropanol, ethyl acetate, acetone, methyl ethyl ketone, trichloromethane, and the like. For certain embodiments, preferably the solvent is isopropanol.

As indicated above, the amorphous glass layer comprising silicon, carbon, and hydrogen is plasma-deposited onto the planarization layer. Coatings are deposited on the planarized polymer film moving on a cooled drum electrode with RF power applied to the drum under vacuum with a very high negative potential on the drum electrode. An organosilicon, such as tetramethylsilane, is introduced without or with some oxygen. Thus, for certain embodiments, including any one of the above embodiments for manufacturing the conformable barrier sheet, plasma-depositing the amorphous glass layer is carried out by introducing a source gas comprising an organosilicon compound and optionally oxygen into a chamber containing the polymeric film coated with the planarization layer. The amorphous glass layer that is formed has a low free volume as a result of the ion bombardment produced by the high substrate bias.

Neither the amorphous glass layer nor the planarization layer introduce any significant opacity to the conformable barrier sheet. For certain embodiments, including any one of the above embodiments, the barrier sheet is sufficiently transparent to visually observe a structure viewed through the barrier sheet. For certain of these embodiments, the barrier sheet is transparent or translucent. For certain of these embodiments, the barrier sheet has an average transmittance of at least 50 percent, preferably at least 70 percent over the visible light wavelength range of 400 nm to 700 nm at a 0 degree angle of incidence (i.e., a 0 degree angle from an axis perpendicular to a major surface of the barrier sheet).

For certain embodiments, including any one of the above embodiment, the barrier sheet absorbs and/or transmits less than 5 percent, preferably less than 2 percent, more preferably less than 1 percent of a pharmaceutically active agent and/or excipient, wherein the pharmaceutically active agent and excipient are dissolved, dispersed, or suspended in a composition contacting and covering a major surface of the barrier sheet. For certain of these embodiments, preferably the barrier sheet absorbs and/or transmits less than 0.5, more preferably less than 0.1 percent of the active agent and/or excipient. For certain embodiments, absorption and/or transmittance of any component in the composition containing the active agent is limited according to any of the above values. For certain of these embodiments, preferably the composition is contacting and covering a major surface of the side of the barrier sheet upon which the amorphous glass layer is deposited.

In one example, the ability of the barrier sheet to resist absorbing a pharmaceutically active agent and/or excipient can be evaluated by measuring the rate at which a test excipient, such as methyl laurate, is taken up by the barrier sheet. The uptake of methyl laurate into the barrier sheet at a timepoint can be modeled by the equation:

$$\text{Mass}(t) - \text{Mass}(0) = [\text{Mass}(\text{sat})][1 - \exp(-k_p t/L)]$$

where, Mass(t) is the mass of the barrier sheet at timepoint t, Mass(0) is the initial mass of the barrier sheet, Mass(sat) is the mass of the barrier sheet once saturated with methyl laurate, $k_p$ is the mass transfer coefficient of methyl laurate in the barrier sheet and characterizes the rate of uptake of methyl laurate into the barrier sheet, t is the time at timepoint t, and L is the thickness of the barrier sheet. For certain embodiments, including any one of the above embodiment, any uptake of methyl laurate into the barrier sheet occurs at a rate wherein the mass transfer coefficient of methyl laurate is less than 0.005 cm/sec, and wherein neat methyl laurate contacts a major surface of the side of the barrier sheet upon which the amorphous glass layer is deposited. The mass transfer coefficient of methyl laurate uptake into a barrier sheet may be conveniently carried out using the test method of Example 9 below.

As indicated above, there is also provided herein a transdermal drug delivery device for delivering a pharmaceutically active agent, which includes the conformable barrier sheet, including any one of the barrier sheet embodiments described above. The reservoir of the device contacts and covers a major surface of the polymeric film or the amorphous glass layer of the conformable barrier sheet. The reservoir contains a pharmaceutically active agent, which can be released to a dermal surface when contacted therewith.

Various transdermal drug delivery device constructions including reservoirs are known and include: devices containing gelled or liquid reservoirs, such as in U. S. Patent No. 4,834,979 (Gale), so-called "reservoir" patches; devices containing matrix reservoirs attached to the skin by an adjacent adhesive layer, such as in U. S. Patent No. 6,004,578 (Lee, et al.), so-called "matrix" patches; and devices containing pressure-sensitive adhesive reservoirs, such as in U. S. Patent No. 6,365,178 (Venkateshwaran et al.), so-called "drug-in-adhesive" patches.

When not in contact with a dermal surface of a subject, for certain embodiments, the reservoir is protected in some manner from the outside environment, such as with a release liner. Any one of these device constructions can be used with the conformable barrier layer described herein to provide the present device. For certain embodiments, including any one of the above transdermal drug delivery device embodiments, the reservoir comprises a pressure sensitive-adhesive.

Exemplary pharmaceutically active agents, also referred to as drugs, that can be included in the reservoir include any substance capable of local or systemic effect when administered to the skin, such as clonidine, estradiol, nicotine, nitroglycerine, scopolamine, rivastigmine, lidocaine, and fentanyl, all of which are commercially available in the form of transdermal devices. Others include antiinflammatory drugs, both steroidal (e.g., hydrocortisone, prednisolone, triamcinolone) and nonsteroidal (e.g., naproxen, piroxicam); bacteriostatic agents (e.g., chlorhexidine, hexylresorcinol); antibacterials (e.g., penicillins such as penicillin V, cephalosporins such as cephalexin, erythromycin, tetracycline, gentamycin, sulfathiazole, nitrofurantoin, and quinolones such as norfloxacin, flumequine, and ibafloxacin); antiprotazoals (e.g., metronidazole); antifungals (e.g., nystatin); coronary vasodilators; calcium channel blockers (e.g., nifedipine, diltiazem); bronchodilators (e.g., theophylline, pirbuterol, salmeterol, isoproterenol); enzyme inhibitors such as collagenase inhibitors, protease inhibitors, elastase inhibitors, lipoxygenase inhibitors (e.g., A64077), and angiotensin converting enzyme inhibitors (e.g., captopril, lisinopril); other antihypertensives (e.g., propranolol); leukotriene antagonists (e.g., ICI204,219); anti-ulceratives such as H2 antagonists; steroidal hormones (e.g., progesterone, testosterone, estradiol); antivirals and/or immunomodulators (e.g., 1-isobutyl-1$H$ imidazo[4,5-$c$]quinolin-4-amine, 1-(2-hydroxy-2-methylpropyl)-1$H$-imidazo[4,5-$c$]quinolin-4-amine, and acyclovir); local anesthetics (e.g., benzocaine, propofol); cardiotonics (e.g., digitalis, digoxin); antitussives (e.g., codeine, dextromethorphan); antihistamines (e.g., diphenhydramine, chlorpheniramine, terfenadine); narcotic analgesics (e.g., morphine, buprenorphine, sentonyl); peptide hormones (e.g., human or animal growth hormones, LHRH); cardioactive products such as atriopeptides; proteinaceous products (e.g., insulin); enzymes (e.g., anti-plaque enzymes, lysozyme, dextranase); antinauseants; anticonvulsants (e.g., carbamazine); immunosuppressives (e.g., cyclosporine); psychotherapeutics (e.g., diazepam); sedatives (e.g., phenobarbital); anticoagulants (e.g., heparin); analgesics (e.g., acetaminophen); antimigraine agents (e.g., ergotamine, melatonin, sumatripan); antiarrhythmic agents (e.g., flecainide); antiemetics (e.g.,metoclopramide, ondansetron); anticancer agents (e.g., methotrexate); neurologic agents such as anxiolytic drugs; hemostatics; anti-obesity agents; and the like, as well as pharmaceutically acceptable salts, esters, and prodrugs thereof. The amount of drug that constitutes a therapeutically effective amount can be readily determined by those skilled in the art with due consideration of the particular drug, the particular carrier, and the desired therapeutic effect.

The reservoir may optionally contain other additives or excipients in addition to the drug and the carrier matrix. Such additives include pharmaceutically acceptable materials that may be used as skin penetration enhancers (i.e., substances that increase the permeation rate of a drug across the skin), thermodynamic activity modifiers (i.e., substances that increase the degree of drug saturation in the reservoir), solubilizers (i.e., substances that effectively solubilize a drug) and/or iontopheresis modifiers or agents. Exemplary materials include $C_8$-$C_{20}$ fatty acids such as isostearic acid, octanoic acid, and oleic acid; $C_8$-$C_{20}$ fatty alcohols such as oleyl alcohol and lauryl alcohol; lower alkyl esters of $C_8$-$C_{20}$ fatty acids such as ethyl oleate, isopropyl myristate, butyl stearate, and methyl laurate; di(lower) alkyl esters of $C_6$-$C_8$ diacids such as diisopropyl adipate; monoglycerides of $C_8$-$C_{20}$ fatty acids such as glyceryl monolaurate; tetraglycol (tetrahydrofurfuryl alcohol polyethylene glycol ether); tetraethylene glycol (ethanol,2,2'-(oxybis(ethylenoxy))diglycol); $C_6$-$C_{20}$ alkyl pyrrolidone carboxylates; polyethylene glycol; propylene glycol; 2-(2-ethoxyethoxy)ethanol; diethylene glycol monomethyl ether; N,N-dimethyldodecylamine-N-oxide and combinations of the foregoing. Alkylaryl ethers of polyethylene oxide, polyethylene oxide monomethyl ethers, polyethylene oxide dimethyl ethers, glycerol, and N-methyl pyrrolidone are also suitable. The terpenes are another useful class of pharmaceutical excipients, including pinene, $d$-limonene, carene, terpineol, terpinen-4-ol, carveol, carvone, pulegone, piperitone, menthone, menthol, neomenthol, thymol, camphor, borneol, citral, ionone, and cineole, alone or in any combination.

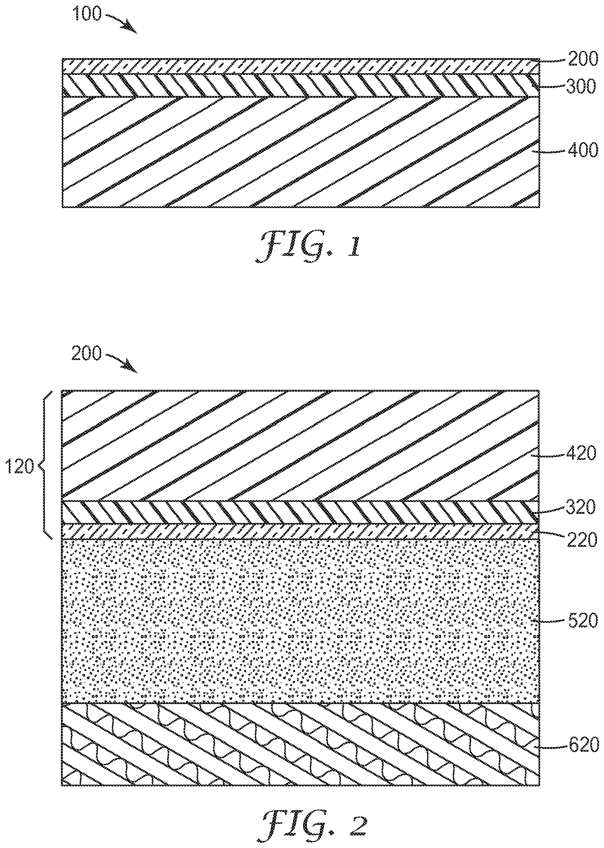

Referring to the drawings, FIG. 1 is an illustration of a conformable barrier sheet provided herein. Conformable barrier sheet 100, shown in cross-section, includes polymeric film 400, having tensile elongation properties described above, which is covered on a major surface with planarization layer 300, which has a thickness and is comprised of a cross-linked polymer as describe above. Amorphous glass layer 200, described above, is deposited on planarization layer 300. It is to be understood that even though a planarization layer and amorphous glass layer are shown on only one side of the polymeric film in the present figures, a planarization layer or other polymeric layer without or with an amorphous glass layer can be applied to the opposing side of the polymeric film. This may balance stress from one side of the film to the other and may provide even greater barrier properties.

FIG. 2 illustrates one embodiment of a transdermal drug delivery device provided herein. Device 200, shown in cross-section, includes conformable barrier sheet 120 with planarization layer 320 disposed on polymeric film 420, and amorphous glass layer 220 deposited on planarization layer 320. Reservoir 520 of device 200 is adjacent to at least a portion of amorphous glass layer 220. As shown, the device further includes release liner 620 covering reservoir 520. Prior to use, release liner 620 protects the surface of reservoir 520, opposed to amorphous glass layer 220, which would otherwise be exposed. In use, release liner 620 is removed and reservoir 520 is placed in contact with a dermal surface.

As shown in FIG. 2, reservoir 520 directly adjoins amorphous glass layer 220 and covers one entire surface of amorphous glass layer 220. The term "adjacent" as stated above, however, should be understood to mean that amorphous glass layer 220 need not be in direct contact with reservoir 520, but may be separated from the reservoir by one or more other layers, for example, an adhesion promoting layer, such as a layer resulting from a treatment selected from the group consisting of air plasma, oxygen plasma, nitrogen plasma, corona, flame, chemical, and a combination thereof. A chemical treatment may include, for example, treating the layer with a coupling agent (e.g., silane coupling agents such as acrylate, methacrylate, and/or epoxy group-containing silanes) or applying a polymerizable layer and forming a polymerized layer (e.g., a polymerized acrylate, methacrylate, or epoxide without or with residual unreacted acrylate, methacrylate, or epoxy groups). Such an adhesion promoting layer or priming layer may also be used between the polymeric film and the planarization layer in any of the embodiments described herein.

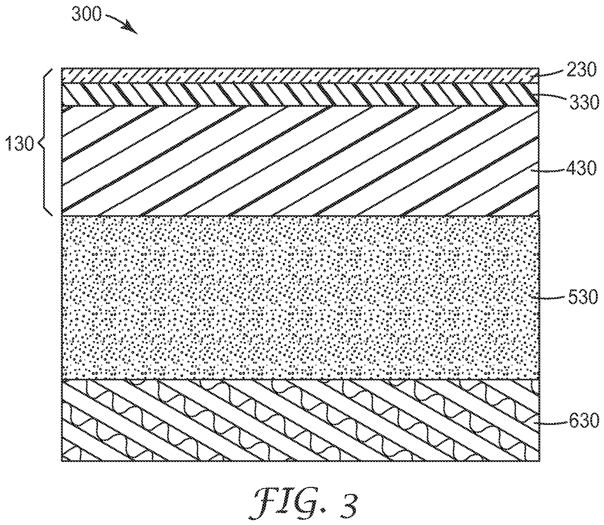

FIG. 3 illustrates another embodiment of a transdermal drug delivery device provided herein. Device 300, shown in cross-section, includes conformable barrier sheet 130 with planarization layer 330 disposed on polymeric film 430, and amorphous glass layer 230 deposited on planarization layer 330. Reservoir 530 of device 300 is adjacent at least a portion of polymeric film 430. As shown, the device further includes release liner 630 covering reservoir 530 as described above in FIG. 2. As shown in FIG. 3, reservoir 530 directly adjoins polymeric film 430. However, as in FIG. 2, polymeric film 430 need not be in direct contact with reservoir 530, but may be separated from the reservoir by one or more other layers, for example, an adhesion promoting layer as described above in FIG. 2.

Although not illustrated, other transdermal drug delivery device constructions are also provided. For example, the reservoir may be partially or completely surrounded by an adhesive for adhering the device to a dermal surface, such as the skin of a subject. In this embodiment, the reservoir contacts the dermal surface, but is not relied upon for keeping the device in place. In such constructions, the adhesive and the reservoir are both adjacent a portion of the conformable barrier sheet, whether it be the polymeric film side or the amorphous glass layer side.

Transdermal drug delivery devices provided herein can be made in the form of an article such as a tape, a patch, a sheet, a dressing, or any other form known to those skilled in the art. Generally, the device will be in the form of a patch of a size suitable to deliver a selected amount of drug to or through the skin.

Generally, the device will have a surface area greater than about 1 cm$^2$, and more typically greater than about 5 cm$^2$. Generally, the device will have a surface area of less than about 100 cm$^2$, preferably less than about 40 cm$^2$.

As indicated above, a release liner that covers and protects the skin-contacting surface of the reservoir prior to use by a patient may be included in the device. Suitable release liners include conventional release liners comprising a known sheet material such as a polyester sheet, a polyethylene sheet, a polypropylene sheet, or a polyethylene-coated paper, coated with a suitable fluoropolymer, silicone, fluorosilicone, inert oil, or wax based coating. The present devices may be packaged individually in a foil-lined pouch for storage and/or provided in a rolled or stacked form suitable for use with a dispensing apparatus.

A method of delivering a drug to a mammal as described above is also provided. A transdermal drug delivery device as described above is provided and placed so that a surface of the reservoir directly adjoins the skin of a mammal. The reservoir is allowed to remain in place for a period of time sufficient to provide a therapeutic effect. The reservoir may be placed in direct contact with the skin surface, such as where the reservoir comprises a pressure-sensitive adhesive. Alternatively, the reservoir may be separated from the skin surface by a membrane or other layer that moderates or controls the delivery of the drug to the skin surface. The length of time that the reservoir remains in a delivering relationship is typically an extended time, preferably from about 12 hours to about 14 days. The length of time that the reservoir remains in a delivering relationship is preferably about 1 day (i.e., daily dosing), about 3 to 4 days (bi-weekly dosing), or about 7 days (weekly dosing).

For certain embodiments, including any one of the above embodiments, preferably the conformable barrier sheet has a moisture vapor transmission rate across the sheet of less than about 10 g/m$^2$/day. For certain of these embodiments, preferably the moisture vapor transmission rate is less than 7.5 g/m$^2$/day, more preferably less than 5 g/m$^2$/day, most preferably less than 4 g/m$^2$/day.

For certain embodiments, including any one of the above embodiments, preferably the conformable barrier sheet has an oxygen transmission rate across the sheet essentially unchanged compared with prior to the barrier sheet being elongated. For certain of these embodiments, the conformable barrier sheet has an oxygen transmission rate of less than about 250, preferably less than 150, more preferably less than 100 cm$^3$/m$^2$/day. For certain embodiments, including any one of the above embodiments, the conformable barrier sheet has undergone a tensile elongation within the range of more than 2 percent to 20 percent, and the barrier sheet has a moisture vapor transmission rate essentially unchanged compared with prior to being elongated. For certain of these embodiments, the elongation is at least 3 percent, at least 4 percent, or at least 5 percent. For certain of these embodiments, the elongation is up to 15 percent or up to 10 percent.

The moisture vapor transmission rate (MVTR) is a measure of the rate at which moisture vapor will diffuse through a film under steady-state conditions, and was herein measured according to ASTM F 1249-90. MVTR was measured by mounting a film sample as a membrane separating two chambers. One chamber contained moist air and the other chamber was slowly purged with dry carrier gas. Any moisture vapor diffusing through the film mixed with the dry carrier gas. The carrier gas was subsequently assayed for moisture vapor concentration. Moisture vapor transmission rates reported in the Examples below were measured using a Permatran-W6 Programmable Water Vapor Permeability Tester (Modern Controls, Inc., MOCON, Minneapolis, MN). Results were provided as a moisture vapor transmission rate across the film in units of g/m$^2$/day. Dry nitrogen was used as the carrier gas. HPLC grade water was used in the wet chamber to produce a 100 % humidity environment. The temperature was set, for example, at 50 °C or 38 °C, thus the MVTR measurements correspond to 50 °C or 38 °C and 100% RH. The diffusion cell area used was 50 cm$^2$.

As indicated above, there is also provided herein a method of protecting an article comprising enveloping the article with a protective covering comprising the conformable high barrier sheet described in any one of the above embodiments. For certain embodiments, the article is any device or part of a device which is sensitive to moisture or oxygen. A portion or all of the protective covering can be the conformable high barrier sheet.

LISTING OF EMBODIMENTS

The following is a listing of certain of the embodiments described herein:

1. A conformable barrier sheet comprising:

a polymeric film having a tensile elongation at least 2 fold greater than a polyethylene terephthalate film of equal dimensions and under an equal load;
a planarization layer; and
a plasma-deposited amorphous glass layer comprising silicon, carbon, and hydrogen;
wherein the planarization layer is disposed on the polymeric film, the amorphous glass layer is deposited on the planarization layer, and
wherein the barrier sheet is sufficiently conformable that after undergoing a tensile elongation within the range of more than 2 % to 20 %, the barrier sheet has a moisture vapor transmission rate essentially unchanged compared with prior to being elongated.

2. The barrier sheet of embodiment 1, wherein the polymeric film has an elongation without breaking of at least 100 % when tested at a thickness of 12.7 to 127 micrometers.

3. The barrier sheet of embodiment 1 or embodiment 2, wherein the planarization layer has a thickness greater than 1.2 micrometers and not greater than 6 micrometers.

4. The barrier sheet of any one of embodiments 1, 2, and 3, wherein the amorphous glass layer on a hydrogen-free basis comprises about 20 to 40 atomic percent silicon and greater than 35 atomic percent carbon, and further comprises less than 45 down to and including zero atomic percent oxygen.

5. The barrier sheet of embodiment 4, wherein the barrier sheet is essentially colorless.

6. The barrier sheet of any one of embodiments 1, 2, and 3, wherein the amorphous glass layer on a hydrogen-free basis comprises about 20 to 40 atomic percent silicon and greater than 50 atomic percent carbon, and further comprises less than 25 down to and including zero atomic percent oxygen.

7. The barrier sheet of embodiment 6, wherein the barrier sheet is a brown color.

8. The barrier sheet of any one of embodiments 1 through 7, wherein the amorphous glass layer has a thickness of 0.05 micrometers to 0.5 micrometers.

9. The barrier sheet of any one of embodiments 1 through 8, wherein the polymeric film is selected from the group consisting of a single layer film and a multilayer film.

10. The barrier sheet of any one of embodiments 1 through 9, wherein the polymeric film comprises a polymer selected from the group consisting high density polyethylene, medium density polyethylene, low density polyethylene, very low density polyethylene, linear low density polyethylene, ultra low density polyethylene, polypropylene, poly(ethylene-co-propylene), poly(ethylene-co-hexene), poly(ethylene-co-octene), poly(ethylene-co-butene), poly(ethylene-co-vinyl acetate), poly(ethylene-co-vinyl alcohol), poly(ethylene-co-acrylic acid), a polyurethane, a blend thereof, and a combination thereof.

11. The barrier sheet of embodiment 10, wherein the polymeric film is selected from the group consisting of poly(ethylene-co-vinyl acetate) with a vinyl acetate content of 2 to 32 percent by weight, a blend of ultra low density polyethylene and linear low density polyethylene, low density polyethylene, and a polyurethane.

12. The barrier sheet of any one of embodiments 1 through 9, wherein the polymeric film comprises a polymer selected from the group consisting high density polyethylene, medium density polyethylene, low density polyethylene, very low density polyethylene, linear low density polyethylene, ultra low density polyethylene, polypropylene, poly(ethylene-co-propylene), poly(ethylene-co-hexene), poly(ethylene-co-octene), poly(ethylene-co-butene), poly(ethylene-co-vinyl acetate), poly(ethylene-co-vinyl alcohol), poly(ethylene-co-acrylic acid), a polyurethane, a thermoplastic polyester elastomer, a blend thereof, and a combination thereof.

13. The barrier sheet of embodiment 12, wherein the polymeric film is selected from the group consisting of poly(ethylene-co-vinyl acetate) with a vinyl acetate content of 2 to 32 percent by weight, a blend of ultra low density polyethylene and linear low density polyethylene, low density polyethylene, a polyurethane, and a thermoplastic polyester elastomer.

14. The barrier sheet of any one of embodiments 1 through 13, wherein the polymeric film has a thickness of no more than about 150 micrometers.

15. The barrier sheet of any one of embodiments 1 through 14, wherein the polymeric film has a thickness of at least about 10 micrometers.

16. The barrier sheet of any one of embodiments 1 through 15, wherein the planarization layer is a cross-linked polymer.

17. The barrier sheet of embodiment 16, wherein the cross-linked polymer comprises a cross-linked multifunctional (meth)acrylate.

18. The barrier sheet of embodiment 17, wherein the multifunctional (meth)acrylate is pentaerythritol tetraacrylate.

19. The barrier sheet of any one of embodiments 1 through 18, wherein the barrier sheet is sufficiently transparent to visually observe a structure viewed through the barrier sheet.

20. The barrier sheet of any one of embodiments 1 through 19, wherein the barrier sheet absorbs or transmits less than 5 percent of a pharmaceutically active agent and/or excipient, wherein the pharmaceutically active agent and excipient are dissolved, dispersed, or suspended in a composition contacting and covering a major surface of the side of the barrier sheet upon which the amorphous glass layer is deposited.

21. The barrier sheet of any one of embodiments 1 through 20, wherein any uptake of methyl laurate into the barrier sheet occurs at a rate wherein the mass transfer coefficient of methyl laurate is less than 0.005 cm/sec, and wherein neat methyl laurate contacts a major surface of the side of the barrier sheet upon which the amorphous glass layer is deposited.

22. A method of manufacturing a conformable barrier sheet, the method comprising:

> providing a polymeric film having an elongation at least 2 fold greater than a polyethylene terephthalate film of equal dimensions and under an equal load;
> forming a planarization layer on the polymeric film; and
> plasma-depositing an amorphous glass layer comprising silicon, carbon, and hydrogen onto the planarization layer;
> wherein the barrier sheet is sufficiently conformable that after undergoing a tensile elongation within the range of more than 2 % to 20 %, the barrier sheet has a moisture vapor transmission rate essentially unchanged compared with prior to being elongated.

23. The method of embodiment 22, wherein plasma-depositing the amorphous glass layer is carried out by introducing a source gas comprising an organosilicon compound and optionally oxygen into a chamber containing the polymeric film coated with the planarization layer.

24. The method of embodiment 22 or embodiment 23, wherein the polymeric film has an elongation without breaking of at least 100 % when tested at a thickness of 12.7 to 127 micrometers.

25. The method of any one of embodiments 22, 23, and 24, wherein the planarization layer has a thickness greater than 1.2 micrometers and not greater than 6 micrometers.

26. The method of any one of embodiments 22 through 25, wherein the amorphous glass layer on a hydrogen-free basis comprises about 20 to 40 atomic percent silicon and greater than 35 atomic percent carbon, and further comprises less than 45 down to and including zero atomic percent oxygen.

27. The method of embodiment 26, wherein the barrier sheet is essentially colorless.

28. The method of any one of embodiments 22 through 25, wherein the amorphous glass layer on a hydrogen-free basis comprises about 20 to 40 atomic percent silicon and greater than 50 atomic percent carbon, and further comprises less than 25 down to and including zero atomic percent oxygen.

29. The method of embodiment 28, wherein the barrier sheet is a brown color.

30. The method of any one of embodiments 22 through 29, wherein the amorphous glass layer has a thickness of 0.05 micrometers to 0.5 micrometers.

31. The method of any one of embodiments 22 through 30, wherein the polymeric film is selected from the group consisting of a single layer film and a multilayer film.

32. The method of any one of embodiments 22 through 31, wherein the polymeric film comprises a polymer selected from the group consisting high density polyethylene, medium density polyethylene, low density polyethylene, very low density polyethylene, linear low density polyethylene, ultra low density polyethylene, polypropylene, poly(ethylene-co-propylene), poly(ethylene-co-hexene), poly(ethylene-co-octene), poly(ethylene-co-butene), poly(ethylene-co-vinyl acetate), poly(ethylene-co-vinyl alcohol), poly(ethylene-co-acrylic acid), a polyurethane, a blend thereof, and a combination thereof.

33. The method of embodiment 32, wherein the polymeric film is selected from the group consisting of poly(ethylene-co-vinyl acetate) with a vinyl acetate content of 2 to 32 percent by weight, a blend of ultra low density polyethylene and linear low density polyethylene, low density polyethylene, and a polyurethane.

34. The method of any one of embodiments 22 through 31, wherein the polymeric film comprises a polymer selected from the group consisting high density polyethylene, medium density polyethylene, low density polyethylene, very low density polyethylene, linear low density polyethylene, ultra low density polyethylene, polypropylene, poly(ethylene-co-propylene), poly(ethylene-co-hexene), poly(ethylene-co-octene), poly(ethylene-co-butene), poly(ethylene-co-vinyl acetate), poly(ethylene-co-vinyl alcohol), poly(ethylene-co-acrylic acid), a polyurethane, a thermoplastic polyester elastomer, a blend thereof, and a combination thereof.

35. The method of embodiment 34, wherein the polymeric film is selected from the group consisting of poly(ethylene-co-vinyl acetate) with a vinyl acetate content of 2 to 32 percent by weight, a blend of ultra low density polyethylene and linear low density polyethylene, low density polyethylene, a polyurethane, and a thermoplastic polyester elastomer.

36. The method of any one of embodiments 22 through 35, wherein the polymeric film has a thickness of no more than 150 micrometers.

37. The method of any one of embodiments 22 through 36, wherein the polymeric film has a thickness of at least 10 micrometers.

38. The method of any one of embodiments 22 through 37, wherein forming the planarization layer on the polymeric film comprises:

coating a cross-linkable composition onto the polymeric film, wherein the cross-linkable composition comprises at least one component having two or more ethylenically unsaturated groups; and
cross-linking the cross-linkable composition.

39. The method of embodiment 38, wherein the at least one component having two or more ethylenically unsaturated groups is a multifunctional (meth)acrylate.

40. The method of embodiment 39, wherein the multifunctional (meth)acrylate is pentaerythritol tetraacrylate.

41. The method of any one of embodiments 22 through 40, wherein the barrier sheet is sufficiently transparent to visually observe a structure viewed through the barrier sheet.

42. The method of any one of embodiments 22 through 41, wherein the barrier sheet absorbs or transmits less than 1 percent of a pharmaceutically active agent and/or excipient, wherein the pharmaceutically active agent and excipient are dissolved, dispersed, or suspended in a composition contacting and covering a major surface of the side of the barrier sheet upon which the amorphous glass layer is deposited.

43. The method of any one of embodiments 22 through 42, wherein any uptake of methyl laurate into the barrier sheet occurs at a rate wherein the mass transfer coefficient of methyl laurate is less than 0.005 cm/sec, and wherein neat methyl laurate contacts a major surface of the side of the barrier sheet upon which the amorphous glass layer is deposited.

44. A transdermal drug delivery device comprising:

a conformable barrier sheet comprising:

a polymeric film having a tensile elongation at least 2 fold greater than a polyethylene terephthalate film of equal dimensions and under an equal load;
a planarization layer; and
a plasma-deposited amorphous glass layer comprising silicon, carbon, and hydrogen;

wherein the planarization layer is disposed on the polymeric film, the amorphous glass layer is deposited on the planarization layer, and

wherein the barrier sheet is sufficiently conformable that after undergoing a tensile elongation within the range of more than 2 % to 20 %, the barrier sheet has a moisture vapor transmission rate essentially unchanged compared with prior to being elongated; and

a reservoir adjoining the polymeric film or the amorphous glass layer, the reservoir comprising a releasably stored dosage of a pharmaceutically active agent.

45. The device of embodiment 44, wherein the polymeric film has an elongation without breaking of at least 100 % when tested at a thickness of 12.7 to 127 micrometers.

46. The device of embodiment 44 or embodiment 45, wherein the planarization layer has a thickness greater than 1.2 micrometers and not greater than 6 micrometers.

47. The device of any one of embodiments 44, 45, and 46, wherein the amorphous glass layer on a hydrogen-free basis comprises about 20 to 40 atomic percent silicon and greater than 35 atomic percent carbon, and further comprises less than 45 down to and including zero atomic percent oxygen.

48. The device of embodiment 47, wherein the barrier sheet is essentially colorless.

49. The device of any one of embodiments 44, 45, and 46, wherein the amorphous glass layer on a hydrogen-free basis comprises about 20 to 40 atomic percent silicon and greater than 50 atomic percent carbon, and further comprises less than 25 down to and including zero atomic percent oxygen.

50. The device of embodiment 49, wherein the barrier sheet is a brown color.

51. The device of any one of embodiments 44 through 50, wherein the amorphous glass layer has a thickness of 0.05 micrometers to 0.5 micrometers.

52. The device of any one of embodiments 44 through 51, wherein the polymeric film is selected from the group consisting of a single layer film and a multilayer film.

53. The device of any one of embodiments 44 through 52, wherein the polymeric film comprises a polymer selected from the group consisting high density polyethylene, medium density polyethylene, low density polyethylene, very low density polyethylene, linear low density polyethylene, ultra low density polyethylene, polypropylene, poly(ethylene-co-propylene), poly(ethylene-co-hexene), poly(ethylene-co-octene), poly(ethylene-co-butene), poly(ethylene-co-vinyl acetate), poly(ethylene-co-vinyl alcohol), poly(ethylene-co-acrylic acid), a polyurethane, a blend thereof, and a combination thereof.

54. The device of embodiment 53, wherein the polymeric film is selected from the group consisting of poly(ethylene-co-vinyl acetate) with a vinyl acetate content of 2 to 32 percent by weight, a blend of ultra low density polyethylene and linear low density polyethylene, low density polyethylene, and a polyurethane.

55. The device of any one of embodiments 44 through 52, wherein the polymeric film comprises a polymer selected from the group consisting high density polyethylene, medium density polyethylene, low density polyethylene, very low density polyethylene, linear low density polyethylene, ultra low density polyethylene, polypropylene, poly(ethylene-co-propylene), poly(ethylene-co-hexene), poly(ethylene-co-octene), poly(ethylene-co-butene), poly(ethylene-co-vinyl acetate), poly(ethylene-co-vinyl alcohol), poly(ethylene-co-acrylic acid), a polyurethane, a thermoplastic polyester elastomer, a blend thereof, and a combination thereof.

56. The device of embodiment 55, wherein the polymeric film is selected from the group consisting of poly(ethylene-co-vinyl acetate) with a vinyl acetate content of 2 to 32 percent by weight, a blend of ultra low density polyethylene and linear low density polyethylene, low density polyethylene, a polyurethane, and a thermoplastic polyester elastomer.

57. The device of any one of embodiments 44 through 56, wherein the polymeric film has a thickness of no more than about 150 micrometers.

58. The device of any one of embodiments 44 through 57, wherein the polymeric film has a thickness of at least about 10 micrometers.

59. The device of any one of embodiments 44 through 58, wherein the planarization layer is a cross-linked polymer.

60. The device of embodiment 59, wherein the cross-linked polymer comprises a cross-linked multifunctional (meth)acrylate.

61. The device of embodiment 60, wherein the multifunctional (meth)acrylate is pentaerythritol tetraacrylate.

62. The device of any one of embodiments 44 through 61, wherein the barrier sheet is sufficiently transparent to visually observe a structure viewed through the barrier sheet.

63. The device of any one of embodiments 44 through 62, wherein the reservoir comprises a pressure sensitive-adhesive.

64. The device of any one of embodiments 44 through 63, wherein the reservoir adjoins the amorphous glass layer.

65. The device of any one of embodiments 44 through 64, wherein the barrier sheet absorbs or transmits less than 1 percent of a pharmaceutically active agent and/or excipient, wherein the pharmaceutically active agent and excipient are dissolved, dispersed, or suspended in a composition contacting and covering a major surface of the side of the barrier sheet upon which the amorphous glass layer is deposited.

66. The device of any one of embodiments 44 through 65, wherein any uptake of methyl laurate into the barrier sheet occurs at a rate wherein the mass transfer coefficient of methyl laurate is less than 0.005 cm/sec, and wherein neat methyl laurate contacts a major surface of the side of the barrier sheet upon which the amorphous glass layer is deposited.

67. The device of any one of embodiments 44 through 63, wherein the reservoir adjoins the polymeric film.

68. The device of any one of embodiments 44 through 63 and 67, wherein the barrier sheet transmits less than 1 percent of a pharmaceutically active agent, wherein the pharmaceutically active agent is dissolved, dispersed, or suspended in a composition contacting and covering a major surface of the polymeric film on side of the barrier sheet opposite the amorphous glass layer.

69. A method of delivering a drug to a mammal comprising;

providing a transdermal drug delivery device according to any one of embodiments 44 through 68;

placing a surface of the reservoir directly adjoining skin of the mammal; and allowing the reservoir to remain directly adjoining the skin for a period of time sufficient to provide a therapeutic effect.

70. The method of embodiment 69, wherein the conformable barrier sheet is sufficiently transparent so that the skin directly adjoining the reservoir can be visually observed to determine the condition of the skin.

71. A method of protecting an article comprising enveloping the article with a protective covering comprising the conformable high barrier sheet of any one of embodiments 1 through 21.

Objects and advantages of the invention are illustrated by the following examples, but the particular materials and amounts thereof recited in these examples, as well as other conditions and details, should not be construed to unduly limit the invention.

EXAMPLES

Example 1

A UV curable polymer solution was made containing 220 grams of pentaerythritol tetraacrylate (commercially available from Sartomer Company, Exton, PA, under the trade designation SR444); 10 grams of the photoinitiator ESACURE KK [commercially available from Lamberti S.p.A, Gallarate, Italy; CAS Name: benzene, (1-methylethenyl)-, homopolymer, Ar-(2-hydroxy-2-methyl-1-oxopropyl) derivs; CAS Registry Number: 163702-01-0] dissolved in 770 grams of isopropyl alcohol. The resulting solution was coated at a web speed of 30 ft/min (9.14 m/min) on a 9 inch (22.86 cm), 76 micrometer caliper, corona treated low density polyethylene vinyl acetate copolymer film (commercially available from the 3M Company, St. Paul, MN, under the trade designation COTRAN 9705) using a slot die coating method with a solution delivery rate to achieve a 2 micrometer dry coating thickness. The coating was dried in-line at 150 °F (65,6 °C) and cured under a nitrogen atmosphere with a UV lamp (commercially available from Fusion UV Systems, Gaithersberg, MD under the trade designation F600 Fusion H LAMP) operating at 100% power, resulting in a dried coating thickness of approximately 2 micrometers.

The polymer coated web described above was loaded into the vacuum chamber of the coating system used to make the plasma coating shown in U.S. Pat. No. 5,888,594 and pumped down to approximately $5 \times 10^{-4}$ Torr ($6.65 \times 10^{-2}$ Pa). Tetramethylsilane (TMS) vapor was continuously introduced into the chamber at a flow rate of 360 sccm and oxygen was continuously introduced into the chamber at a flow rate of 180 sccm. The plasma was initiated and sustained at 2000 watts by applying rf power to the drum electrode. The pressure in the chamber equilibrated at 14 m Torr (1.86 Pa) and the DC self-bias voltage was approximately -520 V. The drum was continuously rotated to provide a web speed of 4 feet/min (1.219 m/min), corresponding to a plasma deposition time of 48 seconds as the web traversed the drum. The resulting plasma coated amorphous glass layer was light tan in color and the thickness was about 0.3 micrometer.

The material described above was portioned into three separate samples each having a diagonal measurement of 10.81 inches (27.46 cm) and an aspect ratio of 3:2. Following ASTM-D638 testing protocols, two of the samples were placed in an extensometer (commercially available from MTS Systems Corporation, Eden Prairie, MN under the trade designation INSIGHT). Without modification of the gripping mechanism, each sample was clamped between two steel shims on the ends traverse with regard to the direction of elongation. The two samples were elongated using 3 pounds (1.36 kg) tension and 6 pounds (2.72 kg) tension, respectively.

A 50 cm$^2$ piece of each sample was placed in a moisture vapor transmission rate (MVTR) test system (commercially available from MOCON Company, Minneapolis, MN under the trade designation PERMATRAN-W, MODEL 700) under the test conditions of 50 °C and 100 percent humidity, in compliance with ASTM F-1249, TAPPI T557, and JIS K-7129 testing protocols. The test results are shown in Table 1.

Table1

| Test | Initial sample (no added tension) | 3 lbs (1.36 kg) tension | 6 lbs (2.72 kg) tension |
|---|---|---|---|
| elongation in inches (% elongation) | none (0%) | 0.025 (0.635 mm) (0.4%) | 0.045 (1.14 mm) (0.8%) |
| MVTR (g/m$^2$/day) | 1.74 | 1.57 | 1.32 |

Example 2

A UV curable polymer solution was made containing 220 grams of pentaerythritol tetraacrylate (commercially available from Sartomer Company, Exton, PA, under the trade designation SR444); 10 grams of the photoinitiator ESACURE KK [commercially available from Lamberti S.p.A, Gallarate, Italy; CAS Name: benzene, (1-methylethenyl)-, homopolymer, Ar-(2-hydroxy-2-methyl-1-oxopropyl) derivs; CAS Registry Number: 163702-01-0] dissolved in 770 grams of isopropyl alcohol. The resulting solution was coated at a web speed of 30 ft/min (9.14 m/min) on a 9 inch (22.86 cm), 76 micrometer caliper, corona treated low density polyethylene vinyl acetate copolymer film (commercially available from the 3M Company, St. Paul, MN, under the trade designation COTRAN 9705) using a slot die coating method with a solution delivery rate to achieve a 2 micrometer dry coating thickness. The coating was dried in-line at 150 °F (65.6 °C) and cured under a nitrogen atmosphere with a UV lamp (commercially available from Fusion UV Systems, Gaithersberg, MD under the trade designation F600 FUSION H LAMP) operating at 100% power, resulting in a dried coating thickness of approximately 2 micrometers.

The polymer coated web described above was loaded into the vacuum chamber of the coating system used to make the plasma coating shown in U.S. Pat. No. 5,888,594 and pumped down to approximately $5 \times 10^{-4}$ Torr (6.65 $\times 10^{-2}$ Pa). Tetramethylsilane (TMS) vapor was introduced continuously into the chamber at a flow rate of 360 sccm and oxygen was continuously introduced into the chamber at a flow rate of 360 sccm. The plasma was initiated and sustained at 2000 watts by applying rf power to the drum electrode. The pressure in the chamber equilibrated at 14 m Torr (1.86 Pa) and the DC self-bias voltage was around -520 V. The drum was continuously rotated to provide a web speed of 4 feet/min (1.219 m/min), corresponding to a plasma deposition time of 48 seconds as the web traversed the drum. The resulting plasma coated amorphous glass layer was colorless and the thickness was about 0.3 micrometer.

The material described above was portioned into two separate samples each having a diagonal measurement of 10.81 inches (27.46 cm) and an aspect ratio of 3:2. Following ASTM-D638 testing protocols, one of the samples was placed in an extensometer (commercially available from MTS Systems Corporation, Eden Prairie, MN under the trade designation INSIGHT). Without modification of the gripping mechanism, the sample was clamped between two steel shims on the ends traverse with regard to the direction of elongation. The sample was elongated using 3 pounds (1.36 kg) tension.

A 50 cm$^2$ piece of each sample was placed in a moisture vapor transmission rate (MVTR) test system (commercially available from the MOCON Company, Minneapolis, MN under the trade designation PERMATRAN-W, MODEL 700) under the test conditions of 50 °C and 100 percent humidity, in compliance with ASTM F-1249, TAPPI T557, and JIS K-7129 testing protocols.

The oxygen transmission rate (OTR) measurement for each sample was conducted following ASTM-D3985 testing protocols. The film sample was mounted as a membrane barrier separating two chambers. One chamber contained oxygen, while the second chamber was slowly purged with nitrogen gas. Oxygen diffused through the film and was mixed with the nitrogen carrier gas. The carrier gas was assayed for oxygen concentration with a coulometric detector. The oxygen transmission rates were measured using an OX-TRAN 1000H instrument (commercially available from MOCON Company, Minneapolis, MN). The test results are shown in Table 2.

Table 2

| Test | Initial sample (no added tension) | 3 lbs (1.36 kg) tension |
|---|---|---|
| elongation in inches (% elongation) | none (0%) | 0.031 (0.79 mm) (0.5%) |
| MVTR (g/m$^2$/day) | 2.2 | 1.79 |
| OTR (cm$^3$/day) | 53.8 | 28.1 |

Example 3

A UV curable polymer solution was made containing 220 grams of pentaerythritol tetraacrylate (commercially available from Sartomer Company, Exton, PA, under the trade designation SR444); 10 grams of the photoinitiator ESACURE KK [commercially available from Lamberti S.p.A, Gallarate, Italy; CAS Name: benzene, (1-methylethenyl)-, homopolymcr, Ar-(2-hydroxy-2-methyl-1-oxopropyl) derivs; CAS Registry Number: 163702-01-0] dissolved in 770 grams of isopropyl alcohol. The resulting solution was coated at a web speed of 30 ft/min (9.14 m/min) on a 9 inch (22.86 cm), 76 micrometer caliper, corona treated low density polyethylene vinyl acetate copolymer film (commercially available from the 3M Company, St. Paul, MN, under the trade designation COTRAN 9705) using a slot die coating method with a solution delivery rate to achieve a 4 micrometer dry coating thickness. The coating was dried in-line at 150 °F (65.6 °C) and cured under a nitrogen atmosphere with a UV lamp (commercially available from Fusion UV Systems, Gaithersberg, MD under the trade designation F600 FUSION H LAMP) operating at 100% power, resulting in a dried coating thickness of approximately 4 micrometers.

The polymer coated web described above was loaded into the vacuum chamber of the coating system used to make the plasma coating shown in U.S. Pat. No. 5,888,594 and pumped down to approximately $5 \times 10^4$ Torr (6.65 $\times 10^{-2}$ Pa). Tetramethylsilane (TMS) vapor was introduced continuously into the chamber at a flow rate of 360 sccm and oxygen was continuously introduced into the chamber at a flow rate of 180 sccm. The plasma was initiated and sustained at 2000 watts by applying rf power to the drum electrode. The pressure in the chamber equilibrated at 14 m Torr (1.86 Pa) and the DC self-bias voltage was around -520 V. The drum was continuously rotated to provide a web speed of 4 feet/min (1.219 m/min), corresponding to a plasma deposition time of 48 seconds as the web traversed the drum. The resulting plasma coated amorphous glass layer was tan in color and the thickness was about 0.3 micrometer.

The material described above was portioned into three separate samples each having a diagonal measurement of 10.81 inches (27.46 cm) and an aspect ratio of 3:2. Following ASTM-D638 testing protocols, two of the samples were placed in an extensometer (commercially available from MTS Systems Corporation, Eden Prairie, MN under the trade designation INSIGHT). Without modification of the gripping mechanism, each sample was clamped between two steel shims on the ends traverse with regard to the direction of elongation. The two samples were elongated using 3 pounds (1.36 kg) tension and 6 pounds (2.72 kg) tension, respectively.

A 50 cm$^2$ piece of each sample was placed in a moisture vapor transmission rate (MVTR) test system (commercially available from MOCON Company, Minneapolis, MN under the trade designation PERMATRAN-W, Model 700) under the test conditions of 50 °C and 100 percent humidity, in compliance with ASTM F-1249, TAPPI T557, and JIS K-7129 testing protocols. The test results are shown in Table 3.

Table 3

| Test | Initial sample (no added tension) | 3 lbs (1.36 kg) tension | 6 lbs (2.72 kg) tension |
|---|---|---|---|
| elongation in inches (% elongation) | none (0%) | 0.033 (084 mm) (0.5%) | 0.065 (1.65 mm) (1.1%) |
| MVTR (g/m$^2$/day) | 4.36 | 4.61 | 4.29 |

Example 4

A UV curable polymer solution was made containing 220 grams of pentaerythritol tetraacrylate (commercially available from Sartomer Company, Exton, PA, under the trade designation SR444); 10 grams of the photoinitiator ESACURE KK [commercially available from Lamberti S.p.A, Gallarate, Italy; CAS Name: benzene, (1-methylethenyl)-, homopolymer, Ar-(2-hydroxy-2-methyl-1-oxopropyl) derivs; CAS Registry Number: 163702-01-0] dissolved in 770 grams of isopropyl alcohol. The resulting solution was coated at a web speed of 30 ft/min (9.14 m/min) on a 9 inch (22.86 cm), 76 micrometer caliper, corona treated low density polyethylene vinyl acetate copolymer film (commercially available from the 3M Company, St. Paul, MN, under the trade designation COTRAN 9705) using a slot die coating method with a solution delivery rate to achieve a 2 micrometer dry coating thickness. The coating was dried in-line at 150 °F (65.6 °C) and cured under a nitrogen atmosphere with a UV lamp (commercially available from Fusion UV Systems, Gaithersberg, MD under the trade designation F600 FUSION H LAMP) operating at 100% power, resulting in a dried coating thickness of approximately 2 micrometers.

The polymer coated web described above was loaded into the vacuum chamber of the coating system used to make the plasma coating shown in U.S. Pat. No. 5,888,594 and pumped down to approximately $5 \times 10^{-4}$ Torr (6.65 $\times 10^{-2}$ Pa). Tetramethylsilane (TMS) vapor was introduced continuously into the chamber at a flow rate of 360 sccm and oxygen was continuously introduced into the chamber at a flow rate of 360 sccm. The plasma was initiated and sustained at 2000 watts by applying rf power to the drum electrode. The pressure in the chamber equilibrated at 14 m Torr (1.86 Pa) and the DC self-bias voltage was around -520 V. The drum was continuously rotated to provide a web speed of 10 feet/min (3.05 m/min), corresponding to a plasma deposition time of 19 seconds as the web traversed the drum. The resulting plasma coated amorphous glass layer was colorless and the thickness was about 0.1 micrometer.

The material described above was portioned into eight separate samples each having a diagonal measurement of 10.81 inches (27.46 cm) and an aspect ratio of 3:2. Following ASTM-D638 testing protocols, seven of the samples were placed in an extensometer (commercially available from MTS Systems Corporation, Eden Prairie, MN under the trade designation INSIGHT). Without modification of the gripping mechanism, each sample was clamped between two steel shims on the ends traverse with regard to the direction of elongation. The seven samples were elongated using one of the following conditions: 3 pounds (1.36 kg) tension, 9 pounds (4.09 kg) tension, 12 pounds (5.45 kg) tension, 15 pounds (6.81 kg) tension, 20 pounds (9.08 kg) tension, 25 pounds (11.35 kg) tension, 33 pounds (14.98 kg) tension.

A 50 $cm^2$ piece of each sample was placed in a moisture vapor transmission rate (MVTR) test system (commercially available from MOCON Company, Minneapolis, MN under the trade designation PERMATRAN-W, MODEL 700) under the test conditions of 50 °C and 100 percent humidity, in compliance with ASTM F-1249, TAPPI T557, and JIS K-7129 testing protocols. The test results are shown in Table 4.

The plasma coated amorphous glass layer was analyzed for relative surface elemental composition using an x-ray photoelectron spectroscopy system (ESCA, commercially available from Physical Electronics, Chanhassen, MN under the trade designation PHI VERSAPROBE 5000). The sample was analyzed in three separate areas and the percent concentration values for carbon, oxygen, and silicon were determined as average values of the three tests (Table 5). The calculations were made ignoring the presence of hydrogen and normalized to 100 percent for the elements present at a detectable level.

Table 4

| Test | Initial sample (no added tension) | 3 lbs (1.36 kg) tension | 9 lbs (4.09 kg) tension | 12 lbs (5.45 kg) tension | 15 lbs (6.81 kg) tension | 20 lbs (9.08 kg) tension | 25 lbs (11.35 kg) tension | 33 lbs (14.98 kg) tension |
|---|---|---|---|---|---|---|---|---|
| elongation in inches (% elongation) | none (0%) | 0.042 (1.07 mm) (0.7%) | 0.113 (2.87 mm) (1.9%) | 0.185 (4.70 mm) (3.1%) | 0.225 (5.72 mm) (3.7%) | 0.291 (7.39 mm) (4.9%) | 0.452 (11.5 mm) (7.5%) | 1.250 (31.8 mm) (20.8%) |
| MVTR ($g/m^2$/day) | 2.93 | 1.82 | 3.29 | 3.10 | 2.87 | 3.33 | 2.76 | 7.13 |

Table 5

| Test | % Carbon | % Oxygen | % Silicon |
|---|---|---|---|
| ESCA | 39 | 39 | 22 |

Example 5

A UV curable polymer solution was made containing 220 grams of pentaerythritol tetraacrylate (commercially available from Sartomer Company, Exton, PA, under the trade designation SR444); 10 grams of the photoinitiator ESACURE KK [commercially available from Lamberti S.p.A, Gallarate, Italy; CAS Name: benzene, (1-methylethenyl)-, homopolymer, Ar-(2-hydroxy-2-methyl-1-oxopropyl) derivs; CAS Registry Number: 163702-01-0] dissolved in 770 grams of isopropyl alcohol. The resulting solution was coated at a web speed of 30 ft/min (9.14 m/min) on a 9 inch (22.86 cm) wide, 76 micrometer caliper, corona treated low density polyethylene vinyl acetate copolymer film (commercially available from the 3M Company, St. Paul, MN, under the trade designation COTRAN 9705) using a slot die coating method with a solution delivery rate to achieve a 4 micrometer dry coating thickness. The coating was dried in-line at 150 °F (65.6 °C) and cured under a nitrogen atmosphere with a UV lamp (commercially available from Fusion UV Systems, Gaithersberg, MD under the trade designation "F600 Fusion H lamp") operating at 100% power, resulting in a dried coating thickness of approximately 4 micrometers.

The polymer coated web described above was loaded into the vacuum chamber of the coating system used to make the plasma coating shown in U.S. Pat. No. 5,888,594 and pumped down to approximately $5 \times 10^{-4}$ Torr ($6.65 \times 10^{-2}$ Pa). Tetramethylsilane (TMS) vapor was introduced continuously into the chamber at a flow rate of 360 sccm. The plasma was initiated and sustained at 2000 watts by applying rf power to the drum electrode. The pressure in the chamber equilibrated at 14 m Torr (1.86 Pa) and the DC self-bias voltage was around -520 V. The drum was continuously rotated to provide a web speed of 10 feet/min (3.05 m/min), corresponding to a plasma deposition time of 19 seconds as the web traversed the drum. The resulting plasma coated amorphous glass layer was gold in color and the thickness was about 0.1 micrometer.

The plasma coated amorphous glass layer was analyzed for relative surface elemental composition using an x-ray photoelectron spectroscopy system (ESCA, commercially available from Physical Electronics, Chanhassen, MN under the trade designation PHI VERSAPROBE 5000). The sample was analyzed in three separate areas and the percent concentration values for carbon, oxygen, and silicon were determined as average values of the three tests (Table 6). The calculations were made ignoring the presence of hydrogen and normalized to 100 percent for the elements present at a detectable level.

Table 6

| Test | % Carbon | % Oxygen | % Silicon |
|------|----------|----------|-----------|
| ESCA | 61 | 19 | 20 |

Example 6

A UV curable polymer solution was made containing 220 grams of pentaerythritol tetraacrylate (commercially available from Sartomer Company, Exton, PA, under the trade designation SR444); 10 grams of the photoinitiator ESACURE KK [commercially available from Lamberti S.p.A, Gallarate, Italy; CAS Name: benzene, (1-methylethenyl)-, homopolymer, Ar-(2-hydroxy-2-methyl-1-oxopropyl) derivs; CAS Registry Number: 163702-01-0] dissolved in 770 grams of isopropyl alcohol. The resulting solution was coated at a web speed of 30 ft/min (9.14 m/min) on a 9 inch (22.86 cm), 76 micrometer caliper, corona treated low density polyethylene film (commercially available from the 3M Company, St. Paul, MN, under the trade designation COTRAN 9720) using a slot die coating method with a solution delivery rate to achieve a 2 micrometer dry coating thickness. The coating was dried in-line at 150 °F (65.6 °C) and cured under a nitrogen atmosphere with a UV lamp (commercially available from Fusion UV Systems, Gaithersberg, MD under the trade designation F600 Fusion H LAMP) operating at 100% power, resulting in a dried coating thickness of approximately 2 micrometer.

The polymer coated web described above was loaded into the vacuum chamber of the coating system used to make the plasma coating shown in U.S. Pat. No. 5,888,594 and pumped down to approximately $5 \times 10^{-4}$ Torr ($6.65 \times 10^{-2}$ Pa). Tetramethylsilane (TMS) vapor was introduced continuously into the chamber at a flow rate of 360 sccm and oxygen was continuously introduced into the chamber at a flow rate of 360 sccm. The plasma was initiated and sustained at 2000 watts by applying rf power to the drum electrode. The pressure in the chamber equilibrated at 14 m Torr (1.86 Pa) and the DC self-bias voltage was around -520 V. The drum was continuously rotated to provide a web speed of 10 feet/min (3.05 m/min), corresponding to a plasma deposition time of 19 seconds as the web traversed the drum. The resulting plasma coated amorphous glass layer was colorless and the thickness was about 0.1 micrometer.

The material described above was portioned into six separate samples each having a diagonal measurement of 10.81 inches (27.46 cm) and an aspect ratio of 3:2. Following ASTM-D638 testing protocols, five of the samples were placed in an extensometer (commercially available from MTS Systems Corporation, Eden Prairie, MN under the trade designation INSIGHT). Without modification of the gripping mechanism, each sample was clamped between two steel shims on the ends traverse with regard to the direction of elongation. The five samples were elongated using one of the following conditions: 3 pounds (1.36 kg) tension, 9 pounds (4.09 kg) tension, 12 pounds (5.45 kg) tension, 15 pounds (6.81 kg) tension, 25 pounds (11.35 kg) tension. The test results are shown in Table 7.

Table 7

| Test | Initial sample (no added tension) | 3 lbs (1.36 kg) tension | 6 lbs (2.72 kg) tension | 9 lbs (4.09 kg) tension | 12 lbs (5.45 kg) tension | 15 lbs (6.81 kg) tension | 20 lbs (9.08 kg) tension | 25 lbs (11.35 kg) tension |
|---|---|---|---|---|---|---|---|---|
| elongation in inches (% elongation) | none (0%) | 0.030 (0.76 mm) (0.5%) | NT | 0.060 (1.52 mm) (1.0%) | 0.095 (2.41 mm) (1.6%) | 0.120 (3.04 mm) (2.0%) | NT | 0.410 (10.4 mm) (6.6%) |
| MVTR (g/m$^2$/day) | 4.22 | 5.33 | NT | 6.12 | NT | 5.34 | NT | 9.9 |

Comparative Example 1

A sample of polyethylene terephthalate (PET) film, 74 micrometer caliper, (commercially available from the 3M Company, St. Paul, MN, under the trade designation SCOTCHPAK 9753) was portioned into six separate samples each having a diagonal measurement of 10.81 inches (27.46 cm) and an aspect ratio of 3:2. Following ASTM-D638 testing protocols, five of the samples were placed in an extensometer (commercially available from MTS Systems Corporation, Eden Prairie, MN under the trade designation INSIGHT). Without modification of the gripping mechanism, each sample was clamped between two steel shims on the ends traverse with regard to the direction of elongation. The five samples were elongated using one of the following conditions: 3 pounds (1.36 kg) tension, 9 pounds (4.09 kg) tension, 12 pounds (5.45 kg) tension, 15 pounds (6.81 kg) tension, and 83 pounds (37.68 kg) tension. The test results are shown in Table 8.

Table 8: Polyethylene Terephthalate Film (SCOTCHPAK 9753)

| Test | Initial sample (no added tension) | 3 lbs (1.36 kg) tension | 6 lbs (2.72 kg) tension | 9 lbs (4.09 kg) tension | 12 lbs (5.45 kg) tension | 15 lbs (6.81 kg) tension | 20 lbs (9.08 kg) tension | 83 lbs (37.68 kg) tension |
|---|---|---|---|---|---|---|---|---|
| elongation in inches (% elongation) | none (0%) | 0.008 (0.2 mm) (0.1%) | NT | 0.023 (0.58 mm) (0.4%) | 0.027 (0.69 mm) (0.4%) | 0.036 (0.91 mm) (06%) | NT | 0.157 (3.99 mm) (2.6%) |

Comparative Example 2

A sample of corona treated, 76 micrometer caliper thick low density polyethylene vinyl acetate copolymer film (commercially available from the 3M Company, St. Paul, MN, under the trade designation COTRAN 9705) was portioned into seven separate samples each having a diagonal measurement of 10.81 inches and an aspect ratio of 3:2. Following ASTM-D638 testing protocols, six of the samples were placed in an extensometer (commercially available from MTS Systems Corporation, Eden Prairie, MN under the trade designation INSIGHT). Without modification of the gripping mechanism, each sample was clamped between two steel shims on the ends traverse with regard to the direction of elongation. The six samples were elongated using one of the following conditions: 3 pounds (1.36 kg) tension, 6 pounds (2.72 kg) tension, 9 pounds (4.09 kg) tension, 12 pounds (5.45 kg) tension, 15 pounds (6.81 kg) tension, 20 pounds (9.08 kg) tension.

The oxygen transmission rate (OTR) of the sample was determined following ASTM-D3985 testing protocols. The COTRAN 9705 film sample was mounted as a membrane barrier separating two chambers. One chamber contained oxygen, while the second chamber was slowly purged with nitrogen gas. Oxygen diffused through the film and was mixed with the nitrogen carrier gas. The carrier gas was assayed for oxygen concentration with a coulometric detector. The oxygen transmission rates were measured using an OX-TRAN 1000H instrument (commercially available from the MOCON Company, Minneapolis, MN).

A 50 cm$^2$ piece of each sample was placed in a moisture vapor transmission rate (MVTR) test system (commercially available from MOCON Company, Minneapolis, MN under the trade designation PERMATRAN-W, MODEL 700) under the test conditions of 50 °C and 100 percent humidity, in compliance with ASTM F-1249, TAPPI T557, and JIS K-7129 testing protocols. The test results are shown in Table 9.

Table 9: COTRAN 9705

| Test | Initial sample (no added tension) | 3 lbs (1.36 kg) tension | 6 lbs (2.72 kg) tension | 9 lbs (4.09 kg) tension | 12 lbs (5.45 kg) tension | 15 lbs (6.81 kg) tension | 20 lbs (6.81 kg) tension |
|---|---|---|---|---|---|---|---|
| elongation in inches (% elongation) | none (0%) | 0.045 (1.14 mm) (0.7%) | 0.092 (2.34 mm) (1.5%) | 0.115 (2.92 mm) (1.9%) | 0.235 (5.97 mm) (3.9%) | 0.335 (8.51 mm) (5.6%) | 0.693 (17.6 mm) (11.5%) |
| MVTR (g/m$^2$/day) | 70 | NT | NT | NT | NT | NT | NT |
| OTR (cm$^3$/m$^2$/day) | 4214.3 | NT | NT | NT | NT | NT | NT |

Comparative Example 3

A UV curable polymer solution was made containing 220 grams of pentaerythritol tetraacrylate (commercially available from Sartomer Company, Exton, PA, under the trade designation SR444); 10 grams of the photoinitiator ESACURE KK [commercially available from Lamberti S.p.A, Gallarate, Italy; CAS Name: benzene, (1-methylethenyl)-, homopolymer, Ar-(2-hydroxy-2-methyl-1-oxopropyl) derivs; CAS Registry Number: 163702-01-0] dissolved in 770 grams of isopropyl alcohol. The resulting solution was coated at a web speed of 30 ft/min (9.14 m/min) on a 9 inch (22.86 cm), 76 micrometer caliper, corona treated low density polyethylene vinyl acetate copolymer film (commercially available from the 3M Company, St. Paul, MN, under the trade designation COTRAN 9705) using a slot die coating method with a solution delivery rate to achieve a 2 micrometer dry coating thickness. The coating was dried in-line at 150 °F (65.6 °C) and cured under a nitrogen atmosphere with a UV lamp (commercially available from Fusion UV Systems, Gaithersberg, MD under the trade designation F600 FUSION H LAMP) operating at 100% power, resulting in a dried coating thickness of approximately 2 micrometers.

A 50 cm$^2$ piece of sample was placed in a moisture vapor transmission rate (MVTR) test system (commercially available from MOCON Company, Minneapolis, MN under the trade designation PERMATRAN-W, MODEL 700) under the test conditions of 50 °C and 100 percent humidity, in compliance with ASTM F-1249, TAPPI T557, and JIS K-7129 testing protocols. The test results are shown in Table 10.

Table 10: COTRAN 9705 with Planarization Layer

| Test | Sample with no added tension |
|---|---|
| MVTR (g/m$^2$/day) | 55 |

Comparative Example 4

A sample of corona treated, 76 micrometer caliper thick low density polyethylene film (commercially available from the 3M Company, St. Paul, MN, under the trade designation COTRAN 9720) was portioned into eight separate samples each having a diagonal measurement of 10.81 inches (27.46 cm) and an aspect ratio of 3:2. Following ASTM-D638 testing protocols, seven of the samples were placed in an extensometer (commercially available from MTS Systems Corporation, Eden Prairie, MN under the trade designation INSIGHT). Without modification of the gripping mechanism, each sample was clamped between two steel shims on the ends traverse with regard to the direction of elongation. The seven samples were elongated using one of the following conditions: 3 pounds (1.36 kg) tension, 6 pounds (2.72 kg) tension, 9 pounds (4.09 kg) tension, 12 pounds (5.45 kg) tension, 15 pounds (6.81 kg) tension, 20 pounds (9.08 kg) tension, 25 pounds (11.35 kg) tension. The test results are shown in Table 11.

Table 11: COTRAN 9720

| Test | Initial sample (no added tension) | 3 lbs (1.36 kg) tension | 6 lbs (2.72 kg) tension | 9 lbs (4.09 kg) tension | 12 lbs (5.45 kg) tension | 15 lbs (6.81 kg) tension | 20 lbs (9.08 kg) tension | 25 lbs (11.35 kg) tension |
|---|---|---|---|---|---|---|---|---|
| elongation in inches (% elongation) | none (0%) | 0.019 (0.48 mm) (0.3%) | 0.046 (1.17 mm) (0.8%) | 0.080 (2.03 mm) (1.3%) | 0.120 (3.05 mm) (2.0%) | 0.169 (4.29 mm) (2.8%) | 0.274 (6.96 mm) (4.6%) | 0.458 (11.63 mm) (7.6%) |
| MVTR (g/m$^2$/day) | 12 | NT | NT | NT | NT | NT | NT | NT |

Example 7

A UV curable polymer solution was made containing 87.5 pounds (39.7 Kg) of pentaerythritol tetraacrylate (commercially available from Sartomer Company, Exton, PA, under the trade designation SR444); 397 grams of the photoinitiator ESACURE KK [commercially available from Lamberti S.p.A, Gallarate, Italy; CAS Name: benzene, (1-methylethenyl)-, homopolymer, Ar-(2-hydroxy-2-methyl-1-oxopropyl) derivs; CAS Registry Number: 163702-01-0] dissolved in 261 pounds (118.4 Kg) of isopropyl alcohol. The resulting solution was coated at a web speed of 150 ft/min (45.7 m/min) on a 49 inch (124.5 cm), 76 micrometer caliper, corona treated low density polyethylene film (commercially available from the 3M Company, St. Paul, MN, under the trade designation COTRAN 9720) using a gravure roll coating method with a solution delivery rate to achieve a 3 micrometer dry coating thickness. The coating was dried in-line at 210 °F (98.9 °C) and cured under a nitrogen atmosphere with a UV lamp (commercially available from Fusion UV Systems, Gaithersberg, MD under the trade designation F600 Fusion H LAMP) operating at 100% power, resulting in a dried coating thickness of approximately 3 micrometer.

The polymer coated web described above was loaded into the vacuum chamber of the coating system used to make the plasma coating shown in U.S. Pat. No. 5,888,594 and pumped down to approximately 5x10$^{-4}$ Torr (6.65 x10$^{-2}$ Pa). Tetramethylsilane (TMS) vapor was introduced continuously into the chamber at a flow rate of 360 sccm and oxygen was continuously introduced into the chamber at a flow rate of 720 sccm. The plasma was initiated and sustained at 6000 watts by applying rf power to the drum electrode. The pressure in the chamber equilibrated at 42 m Torr (5.6 Pa) and the DC self-bias voltage was around -520 V. The drum was continuously rotated to provide a web speed of 5 feet/min (1.5 m/min), corresponding to a plasma deposition time of 19 seconds as the web traversed the drum. The resulting plasma coated amorphous glass layer was colorless and the thickness was about 0.1 micrometer.

A 50 cm$^2$ piece of each sample was placed in a moisture vapor transmission rate (MVTR) test system (commercially available from MOCON Company, Minneapolis, MN under the trade designation PERMATRAN-W, MODEL 700) under the test conditions of 38 °C and 90 percent humidity, in compliance with ASTM F-1249, TAPPI T557, and JIS K-7129 testing protocols. The test results are shown in Table 12.

The oxygen transmission rate (OTR) measurement for each sample was conducted following ASTM-D3985 testing protocols. The film sample was mounted as a membrane barrier separating two chambers. One chamber contained oxygen, while the second chamber was slowly purged with nitrogen gas. Oxygen diffused through the film and was mixed with the nitrogen carrier gas. The carrier gas was assayed for oxygen concentration with a coulometric detector. The oxygen transmission rates were measured using an OX-TRAN 1000H instrument (commercially available from MOCON Company, Minneapolis, MN). The test results are shown in Table 12 for the sample as prepared above (Example 7) and untreated COTRAN 9720 (Comparative Example 4).

Table 12

| Test | COTRAN 9720 (Comparative Example 4) | Example 7 |
|---|---|---|
| MVTR (g/m$^2$/day) | 12 | 2.4 |
| OTR (cm$^3$/m$^2$/day) | 3840 | 502 |

Example 8

A UV curable polymer solution was made containing 87.5 pounds (39.7 Kg) of pentaerythritol tetraacrylate (commercially available from Sartomer Company, Exton, PA, under the trade designation SR444); 397 grams of the photoinitiator ESACURE KK [commercially available from Lamberti S.p.A, Gallarate, Italy; CAS Name: benzene, (1-methylethenyl)-, homopolymer, Ar-(2-hydroxy-2-methyl-1-oxopropyl) derivs; CAS Registry Number: 163702-01-0] dissolved in 261 pounds (118.4 Kg) of isopropyl alcohol. The resulting solution was coated at a web speed of 150 ft/min (45.7 m/min) on a 49 inch (124.5 cm), 76 micrometer caliper, corona treated blended polyethylene film (commercially available from the 3M Company, St. Paul, MN, under the trade designation COTRAN 9722) using a slot die coating method with a solution delivery rate to achieve a 2 micrometer dry coating thickness. The coating was dried in-line at 210 °F (98.9 °C) and cured under a nitrogen atmosphere with a UV lamp (commercially available from Fusion UV Systems, Gaithersberg, MD under the trade designation F600 Fusion H LAMP) operating at 100% power, resulting in a dried coating thickness of approximately 2 micrometer.

The polymer coated web described above was loaded into the vacuum chamber of the coating system used to make the plasma coating shown in U.S. Pat. No. 5,888,594 and pumped down to approximately $5 \times 10^{-4}$ Torr ($6.65 \times 10^{-2}$ Pa). Tetramethylsilane (TMS) vapor was introduced continuously into the chamber at a flow rate of 360 sccm and oxygen was continuously introduced into the chamber at a flow rate of 720 sccm. The plasma was initiated and sustained at 6000 watts by applying rf power to the drum electrode. The pressure in the chamber equilibrated at 42 m Torr (5.6 Pa) and the DC self-bias voltage was around -520 V. The drum was continuously rotated to provide a web speed of 5 feet/min (1.5 m/min), corresponding to a plasma deposition time of 19 seconds as the web traversed the drum. The resulting plasma coated amorphous glass layer was colorless and the thickness was about 0.1 micrometer.

A 50 $cm^2$ piece of each sample was placed in a moisture vapor transmission rate (MVTR) test system (commercially available from MOCON Company, Minneapolis, MN under the trade designation PERMATRAN-W, MODEL 700) under the test conditions of 38 °C and 90 percent humidity, in compliance with ASTM F-1249, TAPPI T557, and JIS K-7129 testing protocols. The test results are shown in Table 13.

The oxygen transmission rate (OTR) measurement for each sample was conducted following ASTM-D3985 testing protocols. The film sample was mounted as a membrane barrier separating two chambers. One chamber contained oxygen, while the second chamber was slowly purged with nitrogen gas. Oxygen diffused through the film and was mixed with the nitrogen carrier gas. The carrier gas was assayed for oxygen concentration with a coulometric detector. The oxygen transmission rates were measured using an OX-TRAN 1000H instrument (commercially available from MOCON Company, Minneapolis, MN). The test results are shown in Table 13 for the sample as prepared above (Example 8) and untreated COTRAN 9722 (film not coated with a planarization layer or an amorphous glass layer).

Table 13

| Test | COTRAN 9722 | Example 8 |
|---|---|---|
| MVTR ($g/m^2$/day) | 6 | 5.7 |
| OTR ($cm^3/m^2$/day) | 6400 | 404 |

Example 9

The rate of uptake of a chemical excipient into films from the previous examples was evaluated. Methyl laurate (available from Penta Manufacturing Company, Fairfield, New Jersey) was used as the model excipient. The uptake experiment involved die cutting a 60 $cm^2$ circular sample of the test film. The mass of the film was weighed. The sample was placed into a Franz permeation cell, characterized by a 5 $cm^2$ opening for the test film, and clamped shut. The film was oriented in the Franz cell such that the plasma coated amorphous glass layer was facing upwards towards the upper reservoir of the Franz cell. The upper reservoir of the cell was filled with approximately 5 mL of methyl laurate, and capped shut. The lower chamber of the Franz cell was left empty. The mass of the test film was measured at selected timepoints of 1 hour, 2 hours, 4 hours, 8 hours, and 24 hours by removing the film from the Franz cell, blotting the film dry, then weighing on a balance. The mass of the test film saturated with methyl laurate was also measured at a timepoint greater than 24 hours. Once the mass was measured, the sample was reinserted into the Franz cell, as described above, and the cell was filled with a fresh 5 mL of methyl laurate. The gain in mass of the test film, attributed to diffusion of methyl laurate into the film, was calculated by taking the difference of the film's mass at each timepoint from its initial mass. For each test film, three replicates were evaluated. These data are shown in Table 14 as an average of the individual replicates and the mass value is reported in milligrams.

The uptake of methyl laurate into the test film at each timepoint was modeled by the equation:

$$\text{Mass(t)} - \text{Mass(0)} = [\text{Mass(sat)}][1 - \exp(-k_p t/L)]$$

where, Mass(t) is the mass of the test film at timepoint t, Mass(0) is the initial mass of the test film, Mass(sat) is the mass of the test film once saturated with methyl laurate, $k_p$ is the mass transfer coefficient of methyl laurate in the test film and characterizes the rate of uptake of methyl laurate into the test film, t is the time at timepoint t, and L is the thickness of the test film. The mass transfer coefficient was determined for each film by using the above equation to fit the methyl laurate uptake data sets (Mass(t)-Mass(0)). The method of least squares was used, by minimizing the sum of squares of the difference of the difference of the predicted mass uptake from the actual mass uptake in Table 14, and using the mass transfer coefficient as the adjustable parameter. The mass transfer coefficients for each test film are shown in Table 14. These data show that the test films with the plasma coated amorphous glass layer each have a mass transfer coefficients less than the corresponding uncoated equivalent.

Table 14. Methyl Laurate Uptake in Test Films

| Time (hr) | CoTran 9705 (Compar. Ex. 2) | Coated CoTran 9705 (Ex. 4) | CoTran 9720 (Compar. Ex. 4) | Coated CoTran 9720 (Ex.6) | CoTran 9722 | Coated CoTran 9722 (Ex. 8) |
|---|---|---|---|---|---|---|
| 0 | 0 mg | 0 mg | 0 mg | 0 mg | 0mg | 0 mg |
| 1 | 8.4 mg | 2.1 mg | 5.4 mg | 1.5 mg | 5.8 mg | 3.2 mg |
| 2 | 9.7 mg | 4.2 mg | 6 mg | 3.5 mg | 7.8 mg | 6.7 mg |
| 4 | 10.6 mg | 8.6 mg | 6.2 mg | 4.4 mg | 8.1 mg | 7.6 mg |
| 8 | 11.4 mg | 11.7 mg | 6.1 mg | 4.9 mg | 8.1 mg | 8.4 mg |
| 24 | 11.9 mg | 13.8 mg | 6 mg | 5.5 mg | 8.1 mg | 8.6 mg |
| Mass (sat) | 12.1 mg | 14.2 mg | 6.1 mg | 5.5 mg | 8.1 mg | 8.6 mg |
| $k_p$ (cm/sec) | 0.0074 | 0.0016 | 0.020 | 0.0031 | 0.010 | 0.0044 |

Various modifications and alterations to the invention will become apparent to those skilled in the art without departing from the scope of the invention as defined by the claims. It should be understood that the invention is not intended to be unduly limited by the embodiments and examples set forth herein and that such examples and embodiments are presented by way of example only with the scope of the invention intended to be limited only by the claims set forth herein.

## Claims

1.   Use of a conformable barrier sheet for packaging of a moisture and/or oxygen sensitive product, or as a backing material in a transdermal drug delivery device, wherein the conformable barrier sheet comprises:

    a polymeric film having a tensile elongation of at least 5 percent when a sample of the film having a 22.9 cm width and 15.2 cm length and having a thickness of 76.2 micrometers is subject to a 11.3 kg load across its full width, following ASTM-D638 testing protocols, wherein the polymeric film is poly(ethylene-co-vinyl acetate) with a vinyl acetate content of 2 to 32 percent by weight;
    a planarization layer having a thickness greater than 1.2 micrometers and not greater than 6 micrometers, wherein the planarization layer is a cross-linked polymer derived from multifunctional (meth)acrylate monomers; and
    a plasma-deposited amorphous glass layer comprising silicon, carbon, and hydrogen;
    wherein the planarization layer is disposed on the polymeric film, and the amorphous glass layer is deposited on the planarization layer, and
    wherein the barrier sheet is sufficiently conformable that after undergoing a tensile elongation within the range of more than 2 % to 10 % following ASTM-D638 testing protocols, the barrier sheet has a moisture vapor transmission rate that has increased less than 2 fold compared with the moisture vapor transmission rate prior to being elongated,
    wherein the moisture vapor transmission rate is measured according to ASTM F 1249-90 under the conditions of 50 °C, 100 % relative humidity and a diffusion cell area of 50 $cm^2$.

2.   The use of claim 1, wherein the amorphous glass layer on a hydrogen-free basis comprises 20 to 40 atomic percent silicon and greater than 35 atomic percent carbon, and further comprises less than 45 down to and including zero atomic percent oxygen.

3. The use of claim 2, wherein the barrier sheet is colorless.

4. The use of any one of claims 1 through 3, wherein the amorphous glass layer has a thickness of 0.05 micrometers to 0.5 micrometers.

5. A transdermal drug delivery device comprising:

a conformable barrier sheet comprising: a polymeric film having a tensile elongation of at least 5 percent when a sample of the film having a 22.9 cm width and 15.2 cm length and having a thickness of 76.2 micrometers is subject to a 11.3 kg load across its full width, following ASTM-D638 testing protocols, wherein the polymeric film is poly(ethylene-co-vinyl acetate) with a vinyl acetate content of 2 to 32 percent by weight;
a planarization layer having a thickness greater than 1.2 micrometers and not greater than 6 micrometers, wherein the planarization layer is a cross-linked polymer derived from multifunctional (meth)acrylate monomers; and
a plasma-deposited amorphous glass layer comprising silicon, carbon, and hydrogen;
wherein the planarization layer is disposed on the polymeric film, and the amorphous glass layer is deposited on the planarization layer, and
wherein the barrier sheet is sufficiently conformable that after undergoing a tensile elongation within the range of more than 2 % to 10 % following ASTM-D638 testing protocols, the barrier sheet has a moisture vapor transmission rate that has increased less than 2 fold compared with prior to being elongated, wherein the moisture vapor transmission rate is measured according to ASTM F 1249-90 under the conditions of 50 °C, 100 % relative humidity and a diffusion cell area of 50 cm$^2$; and
a reservoir adjoining the polymeric film or the amorphous glass layer, the reservoir comprising a releasably stored dosage of a pharmaceutically active agent.

6. The device of claim 5, wherein the amorphous glass layer on a hydrogen-free basis comprises 20 to 40 atomic percent silicon and greater than 35 atomic percent carbon, and further comprises less than 45 down to and including zero atomic percent oxygen.

7. The device of claim 6, wherein the barrier sheet is colorless.

8. The device of any one of claims 5 through 7, wherein the amorphous glass layer has a thickness of 0.05 micrometers to 0.5 micrometers.

9. The device of any one of claims 5 through 8, wherein the device is for use in the delivery of a drug to a mammal, wherein the device is to be employed in a method comprising:

providing the transdermal drug delivery device;
placing a surface of the reservoir directly adjoining skin of the mammal; and
allowing the reservoir to remain directly adjoining the skin for a period of time sufficient to provide a therapeutic effect.

10. Use of a conformable barrier sheet as defined in any one of claims 1 through 4 for the manufacture of a transdermal drug delivery device of any one of claims 5 through 8 for delivering a drug to a mammal by:

providing the transdermal drug delivery device;
placing a surface of the reservoir directly adjoining skin of the mammal; and
allowing the reservoir to remain directly adjoining the skin for a period of time sufficient to provide a therapeutic effect.

**Patentansprüche**

1. Verwendung einer formanpassungsfähigen Sperrfolie zum Verpacken eines feuchtigkeits- und/oder sauerstoffempfindlichen Produkts oder als Trägermaterial in einer transdermalen Medikamentenabgabevorrichtung, wobei die formanpassungsfähige Sperrfolie umfasst:

einen Polymerfilm, der eine Zugdehnung von mindestens 5 Prozent aufweist, wenn eine Probe des Films mit einer Breite von 22,9 cm und einer Länge von 15,2 cm und einer Dicke von 76,2 Mikrometern nach ASTM-D638-Prüfprotokollen einer Belastung von 11,3 kg über ihre gesamte Breite ausgesetzt wird, wobei der Polymerfilm Poly(ethylen-co-vinylacetat) mit einem Vinylacetatgehalt von 2 bis 32 Gewichtsprozent ist;
eine Planarisierungsschicht mit einer Dicke von mehr als 1,2 Mikrometern und nicht mehr als 6 Mikrometern, wobei die Planarisierungsschicht ein vernetztes Polymer ist, das von multifunktionellen (Meth)acrylatmonomeren abgeleitet ist; und
eine plasmaabgeschiedene amorphe Glasschicht, die Silizium, Kohlenstoff und Wasserstoff umfasst;
wobei die Planarisierungsschicht auf dem Polymerfilm angeordnet ist, und die amorphe Glasschicht auf der Planarisierungsschicht abgeschieden ist; und
wobei die Sperrfolie ausreichend formanpassungsfähig ist, so dass sie nach dem Durchführen einer Zugdehnung im Bereich von mehr als 2 % bis 10 % nach ASTM-D638-Prüfprotokollen eine Wasserdampfdurchlässigkeitsrate aufweist, die sich im Vergleich zu der Wasserdampfdurchlässigkeitsrate vor der Dehnung weniger als verdoppelt hat, wobei die Wasserdampfdurchlässigkeitsrate gemäß ASTM F 1249-90 unter den Bedingungen von 50 °C, 100 % relativer Feuchtigkeit und einer Diffusionszellfläche von 50 cm$^2$ gemessen wird.

2.  Verwendung nach Anspruch 1, wobei die amorphe Glasschicht auf wasserstofffreier Basis zu 20 bis 40 Atomprozent Silicium und zu mehr als 35 Atomprozent Kohlenstoff umfasst und ferner zu weniger als 45 bis einschließlich null Atomprozent Sauerstoff umfasst.

3.  Verwendung nach Anspruch 2, wobei die Sperrfolie farblos ist.

4.  Verwendung nach einem der Ansprüche 1 bis 3, wobei die amorphe Glasschicht eine Dicke von 0,05 Mikrometer bis 0,5 Mikrometer aufweist.

5.  Transdermale Medikamentenabgabevorrichtung, die umfasst:

eine formanpassungsfähige Sperrfolie, umfassend: einen Polymerfilm, der eine Zugdehnung von mindestens 5 Prozent aufweist, wenn eine Probe des Films mit einer Breite von 22,9 cm und einer Länge von 15,2 cm und einer Dicke von 76,2 Mikrometern nach ASTM-D638-Prüfprotokollen einer Belastung von 11,3 kg über ihre gesamte Breite ausgesetzt wird, wobei der Polymerfilm Poly(ethylen-co-vinylacetat) mit einem Vinylacetatgehalt von 2 bis 32 Gewichtsprozent ist;
eine Planarisierungsschicht mit einer Dicke von mehr als 1,2 Mikrometern und nicht mehr als 6 Mikrometern, wobei die Planarisierungsschicht ein vernetztes Polymer ist, das von multifunktionellen (Meth)acrylatmonomeren abgeleitet ist; und
eine plasmaabgeschiedene amorphe Glasschicht, die Silizium, Kohlenstoff und Wasserstoff umfasst;
wobei die Planarisierungsschicht auf dem Polymerfilm angeordnet ist und die amorphe Glasschicht auf der Planarisierungsschicht abgeschieden ist; und
wobei die Sperrfolie ausreichend formanpassungsfähig ist, so dass sie nach dem Durchführen einer Zugdehnung im Bereich von mehr als 2 % bis 10 % nach ASTM-D638 Prüfprotokollen eine Wasserdampfdurchlässigkeitsrate aufweist, die sich im Vergleich zu der vor der Dehnung weniger als verdoppelt hat, wobei die Wasserdampfdurchlässigkeitsrate gemäß ASTM F 1249-90 unter den Bedingungen von 50 °C, 100 % relativer Feuchtigkeit und einer Diffusionszellfläche von 50 cm$^2$ gemessen wird; und
ein Reservoir, das an den Polymerfilm oder die amorphe Glasschicht angrenzt, wobei das Reservoir eine freisetzbar gespeicherte Dosierung eines pharmazeutischen Wirkstoffs umfasst.

6.  Vorrichtung nach Anspruch 5, wobei die amorphe Glasschicht auf wasserstofffreier Basis 20 bis 40 Atomprozent Silicium und mehr als 35 Atomprozent Kohlenstoff umfasst, und ferner weniger als 45 bis einschließlich null Atomprozent Sauerstoff umfasst.

7.  Vorrichtung nach Anspruch 6, wobei die Sperrfolie farblos ist.

8.  Vorrichtung nach einem der Ansprüche 5 bis 7, wobei die amorphe Glasschicht eine Dicke von 0,05 Mikrometer bis 0,5 Mikrometer aufweist.

9.  Vorrichtung nach einem der Ansprüche 5 bis 8, wobei die Vorrichtung zur Verwendung bei der Abgabe eines Medikaments an einen Säuger dient, wobei die Vorrichtung in einem Verfahren einzusetzen ist, das umfasst:

das Bereitstellen der transdermalen Medikamentenabgabevorrichtung;
das Anordnen einer Oberfläche des Reservoirs direkt angrenzend an die Haut des Säugers; und
das Zulassen, dass das Reservoir über einen Zeitraum, der für das Bereitstellen einer therapeutischen Wirkung ausreicht, direkt an die Haut angrenzt.

**10.** Verwendung einer formanpassungsfähigen Sperrfolie wie in einem der Ansprüche 1 bis 4 definiert, zur Herstellung einer transdermalen Medikamentenabgabevorrichtung nach einem der Ansprüche 5 bis 8 zur Abgabe eines Medikaments an einen Säuger durch:

das Bereitstellen der transdermalen Medikamentenabgabevorrichtung;
das Anordnen einer Oberfläche des Reservoirs direkt angrenzend an die Haut des Säugers; und
das Zulassen, dass das Reservoir über einen Zeitraum, der für das Bereitstellen einer therapeutischen Wirkung ausreicht, direkt an die Haut angrenzt.

**Revendications**

**1.** Utilisation d'une feuille barrière épousant la forme pour le conditionnement d'un produit sensible à l'humidité et/ou à l'oxygène, ou en tant que matériau de support dans un dispositif d'administration transcutanée de médicament, dans laquelle la feuille barrière épousant la forme comprend :

un film polymère ayant un allongement à la traction d'au moins 5 pour cent lorsqu'un échantillon du film ayant une largeur de 22,9 cm largeur et une longueur de 15,2 cm et ayant une épaisseur de 76,2 micromètres est soumis à une charge de 11,3 kg à travers sa largeur complète, suivant des protocoles d'essai ASTM-D638, dans laquelle le film polymère est du poly(éthylène-co-acétate de vinyle) avec une teneur en acétate de vinyle de 2 à 32 pour cent en poids ;
une couche de planarisation ayant une épaisseur supérieure à 1,2 micromètre et ne dépassant pas 6 micromètres, dans laquelle la couche de planarisation est un polymère réticulé dérivé de monomères (méth)acrylate multifonctionnels ; et
une couche de verre amorphe déposée par plasma comprenant du silicium, du carbone et de l'hydrogène ;
dans laquelle la couche de planarisation est disposée sur le film polymère, et la couche de verre amorphe est déposée sur la couche de planarisation, et
dans laquelle la feuille barrière épouse suffisamment la forme pour qu'après avoir subi un allongement à la traction dans la plage de plus de 2 % à 10 % suivant des protocoles d'essai ASTM-D638, la feuille barrière ait un taux de transmission de vapeur humide qui a augmenté moins de 2 fois par comparaison avec le taux de transmission de vapeur humide avant d'être allongée, dans laquelle le taux de transmission de vapeur humide est mesuré selon ASTM F 1249-90 dans les conditions de 50 °C, 100 % d'humidité relative et une aire de cellule de diffusion de 50 cm$^2$.

**2.** Utilisation selon la revendication 1, dans laquelle la couche de verre amorphe sur une base sans hydrogène comprend 20 à 40 pour cent atomiques de silicium et plus de 35 pour cent atomiques de carbone, et comprend en outre moins de 45 jusqu'à et y compris zéro pour cent atomique d'oxygène.

**3.** Utilisation selon la revendication 2, dans laquelle la feuille barrière est incolore.

**4.** Utilisation selon l'une quelconque des revendications 1 à 3, dans laquelle la couche de verre amorphe a une épaisseur de 0,05 micromètre à 0,5 micromètre.

**5.** Dispositif d'administration transcutanée de médicament, comprenant :

une feuille barrière épousant la forme comprenant : un film polymère ayant un allongement à la traction d'au moins 5 pour cent lorsqu'un échantillon du film ayant une largeur de 22,9 cm largeur et une longueur de 15,2 cm et ayant une épaisseur de 76,2 micromètres est soumis à une charge de 11,3 kg à travers sa largeur complète, suivant des protocoles d'essai ASTM-D638, dans lequel le film polymère est du poly(éthylène-co-acétate de vinyle) avec une teneur en acétate de vinyle de 2 à 32 pour cent en poids ;
une couche de planarisation ayant une épaisseur supérieure à 1,2 micromètre et ne dépassant pas 6 micromètres, dans laquelle la couche de planarisation est un polymère réticulé dérivé de monomères (méth)acrylate multifonctionnels ; et

une couche de verre amorphe déposée par plasma comprenant du silicium, du carbone et de l'hydrogène ; dans lequel la couche de planarisation est disposée sur le film polymère, et la couche de verre amorphe est déposée sur la couche de planarisation, et

dans lequel la feuille barrière épouse suffisamment la forme pour qu'après avoir subi un allongement à la traction dans la plage de plus de 2 % à 10 % suivant des protocoles d'essai ASTM-D638, la feuille barrière ait un taux de transmission de vapeur humide qui a augmenté moins de 2 fois par comparaison avec avant d'être allongée, dans lequel le taux de transmission de vapeur humide est mesuré selon ASTM F 1249-90 dans les conditions de 50 °C, 100 % d'humidité relative et une aire de cellule de diffusion de 50 cm$^2$ ; et

un réservoir attenant au film polymère ou à la couche de verre amorphe, le réservoir comprenant un dosage stocké de manière libérable d'un agent actif sur le plan pharmaceutique.

6. Dispositif selon la revendication 5, dans lequel la couche de verre amorphe sur une base sans hydrogène comprend 20 à 40 pour cent atomiques de silicium et plus de 35 pour cent atomiques de carbone, et comprend en outre moins de 45 jusqu'à et y compris zéro pour cent atomique d'oxygène.

7. Dispositif selon la revendication 6, dans lequel la feuille barrière est incolore.

8. Dispositif selon l'une quelconque des revendications 5 à 7, dans lequel la couche de verre amorphe a une épaisseur de 0,05 micromètre à 0,5 micromètre.

9. Dispositif selon l'une quelconque des revendications 5 à 8, dans lequel le dispositif est destiné à être utilisé pour l'administration d'un médicament à un mammifère, dans lequel le dispositif est destiné à être employé dans un procédé comprenant :

la fourniture du dispositif d'administration transcutanée de médicament ;
la mise en place d'une surface du réservoir directement attenante à la peau du mammifère ; et
le fait de laisser le réservoir rester directement attenant à la peau pendant un laps de temps suffisant pour fournir un effet thérapeutique.

10. Utilisation d'une feuille barrière épousant la forme telle que définie dans l'une quelconque des revendications 1 à 4 pour la fabrication d'un dispositif d'administration transcutanée de médicament selon l'une quelconque des revendications 5 à 8 pour administrer un médicament à un mammifère par :

la fourniture du dispositif d'administration transcutanée de médicament ;
la mise en place d'une surface du réservoir directement attenante à la peau du mammifère ; et
le fait de laisser le réservoir rester directement attenant à la peau pendant un laps de temps suffisant pour fournir un effet thérapeutique.

100 —

200 —
300 —
400 —

*FIG. 1*

200 —

120 {

420 —
320 —
220 —

520 —

620 —

*FIG. 2*

FIG. 3

**EP 2 507 056 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070020451 A1
- US 4834979 A, Gale
- US 6004578 A, Lee
- US 6365178 B, Venkateshwaran
- US 5888594 A

**Non-patent literature cited in the description**

- *CHEMICAL ABSTRACTS,* 163702-01-0